# EUROPEAN PATENT SPECIFICATION

(11) **EP 3 497 130 B1**
(45) Date of publication and mention of the grant of the patent: **27.10.2021**
(21) Application number: 17755458.1
(22) Date of filing: 11.08.2017
(51) Int. Cl.: C07K 16/28, A61K 38/17, C07K 14/71, A61P 35/00, A61K 31/282, A61K 31/513, A61K 39/395

(54) **COMBINATION THERAPY FOR CANCER**
KOMBINATIONSTHERAPIE GEGEN KREBS
POLYTHÉRAPIE CONTRE LE CANCER

(30) Priority: 12.08.2016 US 201662374621 P
(43) Date of publication of application: 19.06.2019
(73) Proprietor: Merck Patent GmbH, 64293 Darmstadt (DE)
(72) Inventor: LO, Kin-Ming, Lexington Massachusetts 02420 (US); LAN, Yan, Belmont Massachusetts 02478 (US)
(74) Representative: Mewburn Ellis LLP
(86) International application number: PCT/EP2017/070513
(87) International publication number: WO 2018/029367

(56) References cited:
- WO-A1-2013/079174
- WO-A1-2013/181452
- WO-A2-2015/118175
- R. STEWART ET AL: "Identification and Characterization of MEDI4736, an Antagonistic Anti-PD-L1 Monoclonal Antibody", CANCER IMMUNOLOGY RESEARCH, vol. 3, no. 9, 1 September 2015 (2015-09-01), pages 1052-1062, XP55247766, US ISSN: 2326-6066, DOI: 10.1158/2326-6066.CIR-14-0191

## Description

### FIELD OF THE INVENTION

This invention relates generally to a combination therapy for the treatment of cancer, particularly to a combination of (i) a bifunctional molecule comprising a TGFβRII or fragment thereof capable of binding TGFβ and an antibody, or antigen binding fragment thereof, that binds to an immune checkpoint protein, such as Programmed Death Ligand 1 (PD-L1) and (ii) radiation. In certain embodiments of the invention, the combination therapy provides for a synergistic anti-cancer effect.

### BACKGROUND

In cancer treatment, it has long been recognized that chemotherapy is associated with high toxicity and can lead to emergence of resistant cancer cell variants. Most chemotherapeutic agents cause undesirable side effects including cardiac and renal toxicity, alopecia, nausea and vomiting. Radiation therapy is also used in cancer treatment. Such treatment uses high-energy particles or waves, such as x-rays, gamma rays, electron beams, or protons, to destroy or damage cancer cells. Unlike chemotherapy, which exposes the whole body to cancer-fighting drugs, radiation therapy is more commonly a local treatment. However, it is difficult to selectively administer therapeutic radiation only to the abnormal tissue and, thus, normal tissue near the abnormal tissue is also exposed to potentially damaging doses of radiation throughout treatment.

Cancer immunotherapy is a new paradigm in cancer treatment that instead of targeting cancer cells focuses on the activation of the immune system. Its principle is to rearm the host's immune response, especially the adaptive T cell response, to provide immune surveillance to kill the cancer cells, in particular, the minimal residual disease that has escaped other forms of treatment, hence achieving long-lasting protective immunity.

FDA approval of the anti-CTLA-4 antibody ipilimumab for the treatment of melanoma in 2011 ushered in a new era of cancer immunotherapy. The demonstration that anti-PD-1 or anti-PD-L1 therapy induced durable responses in melanoma, kidney, and lung cancer in clinical trials further signify its coming of age (Pardoll, D.M., Nat Immunol. 2012; 13:1129-32). However, ipilimumab therapy is limited by its toxicity profile, presumably because anti-CTLA-4 treatment, by interfering with the primary T cell inhibitory checkpoint, can lead to the generation of new autoreactive T cells. While inhibiting the PD-L1/PD-1 interaction results in dis-inhibiting existing chronic immune responses in exhausted T cells that are mostly antiviral or anticancer in nature (Wherry, E.J., Nat Immunol. 2011; 12:492-9), anti-PD-1 therapy can nevertheless sometimes result in potentially fatal lung-related autoimmune adverse events. Another approach is to co-target TGFβ and PD_L1 with the anti PD-L1/TGFβ Trap fusion protein consisting of a human TGFβRII and anti human PD-L1 antibody, shown to result in synergistic inhibition of tumor in WO 2015/118175 when compared with the separate administration of the two proteins. Despite the promising clinical activities of anti-PD1 and anti-PD-L1 so far, increasing the therapeutic index, either by increasing therapeutic activity or decreasing toxicity, or both, remains a central goal in the development of immunotherapeutics.

### SUMMARY OF THE INVENTION

The present invention is based on the discovery of a combination therapy for cancer that includes administration of a bifunctional protein containing at least a portion of TGFβ Receptor II (TGFβRII) that is capable of binding TGFβ and an antibody, or antigen-binding fragment, that binds to an immune checkpoint protein such as human protein Programmed Death Ligand 1 (PD-L1). The combination therapy also includes administration of an anti-cancer therapeutic agent such as, for example, radiation, chemotherapeutic agents, a biologic and/or a vaccine. The combination therapy exhibits a synergistic effect as compared to the effect of administering the individual agents separately. The invention is defined in the claims set out herein.

The disclosure features a method of treating cancer in a subject that includes (i) administration of a bifunctional protein comprising a human TGFβRII, or a fragment thereof capable of binding TGFβ (*e.g.*, a soluble fragment), and an antibody, or an antigen-binding fragment thereof, that binds PD-L1 (*e.g.*, any of the antibodies or antibody fragments described herein); and (ii) administration of at least one additional second anti-cancer therapeutic agent.

In certain disclosure herein, the combination treatment method of the disclosure features the use of a polypeptide including (a) at least a variable domain of a heavy chain of an antibody that binds PD-L1 (*e*.*g*., amino acids 1-120 of SEQ ID NO: 2); and (b) human TGFβRII, or a soluble fragment thereof capable of binding TGFβ (*e.g.*, a human TGFβRII extra-cellular domain (ECD), amino acids 24-159 of SEQ ID NO: 9, or any of those described herein) in combination with at least one additional anti-cancer therapeutic agent. The polypeptide may further include an amino acid linker connecting the C-terminus of the variable domain to the N-terminus of the human TGFβRII or soluble fragment thereof capable of binding TGFβ. The polypeptide may include the amino acid sequence of SEQ ID NO: 3 or an amino acid sequence substantially identical to SEQ ID NO: 3. The antibody fragment may be an scFv, Fab, F(ab')₂, or Fv fragment.

In certain disclosure herein, the protein or polypeptide includes an antibody or antigen-binding fragment thereof that includes SEQ ID NO: 2 and human TGFβRII. The antibody may optionally include a modified constant region (e.g., any described herein, including a C-terminal Lys→Ala substitution, a mutation of the Leu-Ser-Leu-Ser (SEQ ID NO: 19) sequence to Ala-Thr-Ala-Thr (SEQ ID NO: 20), or a hybrid constant region including an IgG1 hinge region and an IgG2 CH2 domain).

In certain disclosure herein, the protein or polypeptide includes an antibody or antigen-binding fragment thereof that includes SEQ ID NO: 2 and a fragment of human TGFβRII capable of binding TGFβ (*e.g.*, a soluble fragment). The antibody may optionally include a modified constant region (e.g., any described herein, including a C-terminal Lys→Ala substitution, a mutation of the Leu-Ser-Leu-Ser (SEQ ID NO: 19) sequence to Ala-Thr-Ala-Thr (SEQ ID NO: 20), or a hybrid constant region including an IgG1 hinge region and an IgG2 CH2 domain).

In certain disclosure herein, the protein or polypeptide includes an antibody or antigen-binding fragment thereof that includes SEQ ID NO: 2 and a human TGFβRII ECD. The antibody may include a modified constant region (e.g., any described herein, including a C-terminal Lys→Ala substitution, a mutation of the Leu-Ser-Leu-Ser (SEQ ID NO: 19) sequence to Ala-Thr-Ala-Thr (SEQ ID NO: 20), or a hybrid constant region including an IgG1 hinge region and an IgG2 CH2 domain).

In certain disclosure herein, the protein or polypeptide includes an antibody or antigen-binding fragment thereof that includes amino acids 1-120 of SEQ ID NO: 2 and human TGFβRII. The antibody may include a modified constant region (e.g., any described herein, including a C-terminal Lys→Ala substitution, a mutation of the Leu-Ser-Leu-Ser (SEQ ID NO: 19) sequence to Ala-Thr-Ala-Thr (SEQ ID NO: 20), or a hybrid constant region including an IgG1 hinge region and an IgG2 CH2 domain).

In certain disclosure herein, the protein or polypeptide includes an antibody or antigen-binding fragment thereof that includes amino acids 1-120 of SEQ ID NO: 2 and a fragment of human TGFβRII capable of binding TGFβ (*e.g.*, a soluble fragment). The antibody may include a modified constant region (e.g., any described herein, including a C-terminal Lys→Ala substitution, a mutation of the Leu-Ser-Leu-Ser (SEQ ID NO: 19) sequence to Ala-Thr-Ala-Thr (SEQ ID NO: 20), or a hybrid constant region including an IgG1 hinge region and an IgG2 CH2 domain).

In certain disclosure herein, the protein or polypeptide includes an antibody or antigen-binding fragment thereof that includes amino acids 1-120 of SEQ ID NO: 2 and a human TGFβRII ECD. The antibody may include a modified constant region (e.g., any described herein, including a C-terminal Lys→Ala substitution, a mutation of the Leu-Ser-Leu-Ser (SEQ ID NO: 19) sequence to Ala-Thr-Ala-Thr (SEQ ID NO: 20), or a hybrid constant region including an IgG1 hinge region and an IgG2 CH2 domain).

In certain disclosure herein, the protein or polypeptide includes an antibody or antigen-binding fragment thereof that includes the hypervariable regions present in SEQ ID NO: 2 and human TGFβRII. The antibody may include a modified constant region (e.g., any described herein, including a C-terminal Lys→Ala substitution, a mutation of the Leu-Ser-Leu-Ser (SEQ ID NO: 19) sequence to Ala-Thr-Ala-Thr (SEQ ID NO: 20), or a hybrid constant region including an IgG1 hinge region and an IgG2 CH2 domain).

In certain disclosure herein, the protein or polypeptide includes an antibody or antigen-binding fragment thereof that includes the hypervariable regions present in SEQ ID NO: 2 and a fragment of human TGFβRII capable of binding TGFβ (*e.g.*, a soluble fragment). The antibody may include a modified constant region (e.g., any described herein, including a C-terminal Lys→Ala substitution, a mutation of the Leu-Ser-Leu-Ser (SEQ ID NO: 19) sequence to Ala-Thr-Ala-Thr (SEQ ID NO: 20), or a hybrid constant region including an IgG1 hinge region and an IgG2 CH2 domain).

In certain disclosure herein, the protein or polypeptide includes an antibody or antigen-binding fragment thereof that includes the hypervariable regions present in SEQ ID NO: 2 and a human TGFβRII ECD. The antibody may include a modified constant region (*e.g.*, any described herein, including a C-terminal Lys→Ala substitution, a mutation of the Leu-Ser-Leu-Ser (SEQ ID NO: 19) sequence to Ala-Thr-Ala-Thr (SEQ ID NO: 20), or a hybrid constant region including an IgG1 hinge region and an IgG2 CH2 domain).

In certain disclosure herein, the protein or polypeptide includes an antibody or antigen-binding fragment thereof that includes SEQ ID NO: 12 and human TGFβRII. The antibody may include a modified constant region (e.g., any described herein, including a C-terminal Lys→Ala substitution, a mutation of the Leu-Ser-Leu-Ser (SEQ ID NO: 19) sequence to Ala-Thr-Ala-Thr (SEQ ID NO: 20), or a hybrid constant region including an IgG1 hinge region and an IgG2 CH2 domain).

In certain disclosure herein, the protein or polypeptide includes an antibody or antigen-binding fragment thereof that includes SEQ ID NO: 12 and a fragment of human TGFβRII capable of binding TGFβ (*e.g.*, a soluble fragment). The antibody may include a modified constant region (e.g., any described herein, including a C-terminal Lys→Ala substitution, a mutation of the Leu-Ser-Leu-Ser (SEQ ID NO: 19) sequence to Ala-Thr-Ala-Thr (SEQ ID NO: 20), or a hybrid constant region including an IgG1 hinge region and an IgG2 CH2 domain).

In certain disclosure herein, the protein or polypeptide includes an antibody or antigen-binding fragment thereof that includes SEQ ID NO: 12 and a human TGFβRII ECD. The antibody may include a modified constant region (e.g., any described herein, including a C-terminal Lys→Ala substitution, a mutation of the Leu-Ser-Leu-Ser (SEQ ID NO: 19) sequence to Ala-Thr-Ala-Thr (SEQ ID NO: 20), or a hybrid constant region including an IgG1 hinge region and an IgG2 CH2 domain).

In certain disclosure herein, the protein or polypeptide includes an antibody or antigen-binding fragment thereof that includes the hypervariable regions present in SEQ ID NO: 12 and human TGFβRII. The antibody may include a modified constant region (e.g., any described herein, including a C-terminal Lys→Ala substitution, a mutation of the Leu-Ser-Leu-Ser (SEQ ID NO: 19) sequence to Ala-Thr-Ala-Thr (SEQ ID NO: 20), or a hybrid constant region including an IgG1 hinge region and an IgG2 CH2 domain).

In certain disclosure herein, the protein or polypeptide includes an antibody or antigen-binding fragment thereof that includes the hypervariable regions present in SEQ ID NO: 12 and a fragment of human TGFβRII capable of binding TGFβ (*e.g.*, a soluble fragment). The antibody may include a modified constant region (e.g., any described herein, including a C-terminal Lys→Ala substitution, a mutation of the Leu-Ser-Leu-Ser (SEQ ID NO: 19) sequence to Ala-Thr-Ala-Thr (SEQ ID NO: 20), or a hybrid constant region including an IgG1 hinge region and an IgG2 CH2 domain).

In certain disclosure herein, the protein or polypeptide includes an antibody or antigen-binding fragment thereof that includes the hypervariable regions present in SEQ ID NO: 12 and a human TGFβRII ECD. The antibody may include a modified constant region (*e.g.*, any described herein, including a C-terminal Lys→Ala substitution, a mutation of the Leu-Ser-Leu-Ser (SEQ ID NO: 19) sequence to Ala-Thr-Ala-Thr (SEQ ID NO: 20), or a hybrid constant region including an IgG1 hinge region and an IgG2 CH2 domain).

In certain disclosure herein, the protein or polypeptide includes an antibody or antigen-binding fragment thereof that includes SEQ ID NO: 14 and human TGFβRII. The antibody may include a modified constant region (e.g., any described herein, including a C-terminal Lys→Ala substitution, a mutation of the Leu-Ser-Leu-Ser (SEQ ID NO: 19) sequence to Ala-Thr-Ala-Thr (SEQ ID NO: 20), or a hybrid constant region including an IgG1 hinge region and an IgG2 CH2 domain).

In certain disclosure herein, the protein or polypeptide includes an antibody or antigen-binding fragment thereof that includes SEQ ID NO: 14 and a fragment of human TGFβRII capable of binding TGFβ (*e.g.*, a soluble fragment). The antibody may include a modified constant region (e.g., any described herein, including a C-terminal Lys→Ala substitution, a mutation of the Leu-Ser-Leu-Ser (SEQ ID NO: 19) sequence to Ala-Thr-Ala-Thr (SEQ ID NO: 20), or a hybrid constant region including an IgG1 hinge region and an IgG2 CH2 domain).

In certain disclosure herein, the protein or polypeptide includes an antibody or antigen-binding fragment thereof that includes SEQ ID NO: 14 and a human TGFβRII ECD. The antibody may include a modified constant region (e.g., any described herein, including a C-terminal Lys→Ala substitution, a mutation of the Leu-Ser-Leu-Ser (SEQ ID NO: 19) sequence to Ala-Thr-Ala-Thr (SEQ ID NO: 20), or a hybrid constant region including an IgG1 hinge region and an IgG2 CH2 domain).

The disclosure also provides for the use, in the combination therapy of the disclosure, of a protein including the polypeptide described above and at least a variable domain of a light chain of an antibody which, when combined with the polypeptide, forms an antigen-binding site that binds PD-L1. The protein may include (a) two polypeptides, each having an amino acid sequence consisting of the amino acid sequence of SEQ ID NO: 3, and (b) two additional polypeptides each having an amino acid sequence consisting of the amino acid sequence of SEQ ID NO: 1.

The disclosure features a combination therapy for treatment of cancer which comprises the administration of a protein described above, in combination with administration of one or more additional anti-cancer therapeutic agents for use in treating cancer or for use in inhibiting tumor growth. The one or more additional anti-cancer therapeutic agents include radiation, a chemotherapeutic agent, a biologic, and/or a vaccine.

The cancer or tumor may be selected from the group consisting of colorectal, breast, ovarian, pancreatic, gastric, prostate, renal, cervical, myeloma, lymphoma, leukemia, thyroid, endometrial, uterine, bladder, neuroendocrine, head and neck, liver, nasopharyngeal, testicular, small cell lung cancer, non-small cell lung cancer, melanoma, basal cell skin cancer, squamous cell skin cancer, dermatofibrosarcoma protuberans, Merkel cell carcinoma, glioblastoma, glioma, sarcoma, mesothelioma, and myelodysplastic syndromes.

The disclosure also features a combination therapy method of inhibiting tumor growth or treating cancer. The method includes exposing the tumor to a protein described above. The method further includes exposing the tumor to radiation and or administration of a chemotherapeutic, a biologic, or a vaccine. In certain disclosure herein, the tumor or cancer is selected from the group consisting of colorectal, breast, ovarian, pancreatic, gastric, prostate, renal, cervical, myeloma, lymphoma, leukemia, thyroid, endometrial, uterine, bladder, neuroendocrine, head and neck, liver, nasopharyngeal, testicular, small cell lung cancer, non-small cell lung cancer, melanoma, basal cell skin cancer, squamous cell skin cancer, dermatofibrosarcoma protuberans, Merkel cell carcinoma, glioblastoma, glioma, sarcoma, mesothelioma, and myelodysplastic syndromes.

By "TGFβRII" or "TGFβ Receptor II" is meant a polypeptide having the wild-type human TGFβ Receptor Type 2 Isoform A sequence (e.g., the amino acid sequence of NCBI Reference Sequence (RefSeq) Accession No. NP_001020018 (SEQ ID NO: 8)), or a polypeptide having the wild-type human TGFβ Receptor Type 2 Isoform B sequence (e.g., the amino acid sequence of NCBI RefSeq Accession No. NP_003233 (SEQ ID NO: 9)) or having a sequence substantially identical the amino acid sequence of SEQ ID NO: 8 or of SEQ ID NO: 9. The TGFβRII may retain at least 0.1%, 0.5%, 1%, 5%, 10%, 25%, 35%, 50%, 75%, 90%, 95%, or 99% of the TGFβ-binding activity of the wild-type sequence. The polypeptide of expressed TGFβRII lacks the signal sequence.

By a "fragment of TGFβRII capable of binding TGFβ" is meant any portion of NCBI RefSeq Accession No. NP_001020018 (SEQ ID NO: 8) or of NCBI RefSeq Accession No. NP_003233 (SEQ ID NO: 9), or a sequence substantially identical to SEQ ID NO: 8 or SEQ ID NO: 9 that is at least 20 (*e.g.*, at least 30, 40, 50, 60, 70, 80, 90, 100, 110, 120, 130, 140, 150, 160, 175, or 200) amino acids in length that retains at least some of the TGFβ-binding activity (e.g., at least 0.1%, 0.5%, 1%, 5%, 10%, 25%, 35%, 50%, 75%, 90%, 95%, or 99%) of the wild-type receptor or of the corresponding wild-type fragment. Typically such fragment is a soluble fragment. An exemplary such fragment is a TGFβRII extra-cellular domain having the sequence of SEQ ID NO: 10.

By "substantially identical" is meant a polypeptide exhibiting at least 50%, desirably 60%, 70%, 75%, or 80%, more desirably 85%, 90%, or 95%, and most desirably 99% amino acid sequence identity to a reference amino acid sequence. The length of comparison sequences will generally be at least 10 amino acids, desirably at least 15 contiguous amino acids, more desirably at least 20, 25, 50, 75, 90, 100, 150, 200, 250, 300, or 350 contiguous amino acids, and most desirably the full-length amino acid sequence.

By "patient" is meant either a human or non-human animal (*e.g.*, a mammal).

By "treating" a disease, disorder, or condition (*e.g.*, a cancer) in a patient is meant reducing at least one symptom of the disease, disorder, or condition by administrating a therapeutic agent to the patient.

By "cancer" is meant a collection of cells multiplying in an abnormal manner.

Other embodiments and details of the invention are presented herein below.

### BRIEF DESCRIPTION OF THE DRAWINGS

**FIG. 1** is a schematic drawing of an anti-PD-L1/TGFβ Trap molecule comprising one anti-PD-L1 antibody fused to two extracellular domain (ECD) of TGFβ Receptor II via a (Gly₄Ser)₄Gly linker.
**FIG. 2** is a table summarizing the study design for study "TI13-027: Combination of Anti-PD-L1/TGFβ Trap with 5-FU and Oxaliplatin Therapy in MC38 Tumor Model in C57B/L6 Wild Type Mice" where group and treatment is N=10 mice/group.
**FIG. 3** is a table summarizing the study design for study "TI14-012: Combination of Anti-PD-L1/TGFβ Trap with 5-FU and Oxaliplatin Therapy in MC38 Tumor Model in B Cell Deficient Mice" where group and treatment is N=10 mice/group.

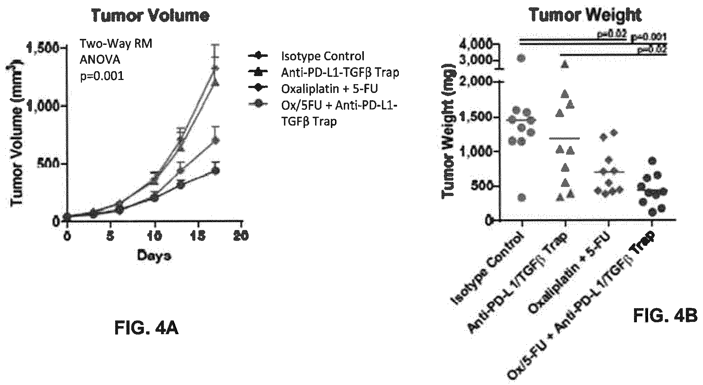

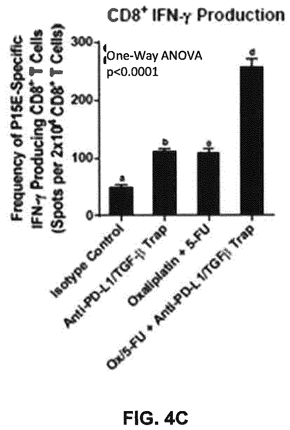

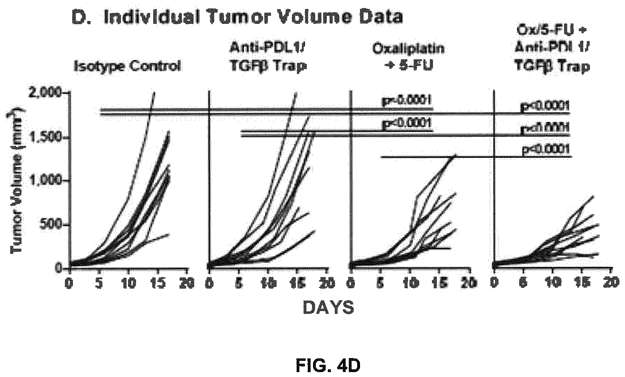

**FIG. 4A-4D** are a series of graphs showing that the Oxaliplatin/5-FU and anti-PD-L1/TGFβ trap combination enhances tumor growth inhibition and tumor-reactive CD8⁺ T cell responses (C57BL/6 Mice; Study TI13-027). FIG. 4A and FIG. 4D Tumor volumes were measured twice per week throughout the study period. Tumor volume data was log transformed and a two-way, repeated measure ANOVA was performed. FIG. 4B. Tumor weight data was evaluated with one-way ANOVA. FIG. 4C. The frequency of IFN-γ producing, P15E-specific CD8⁺ T cells was quantified by ELISpot assay. ELISpot data was evaluated by one-way ANOVA. All ANOVA included Tukey's correction for multiple comparisons to measure statistical differences between treatment groups. p<0.05 was determined to be statistically significant.

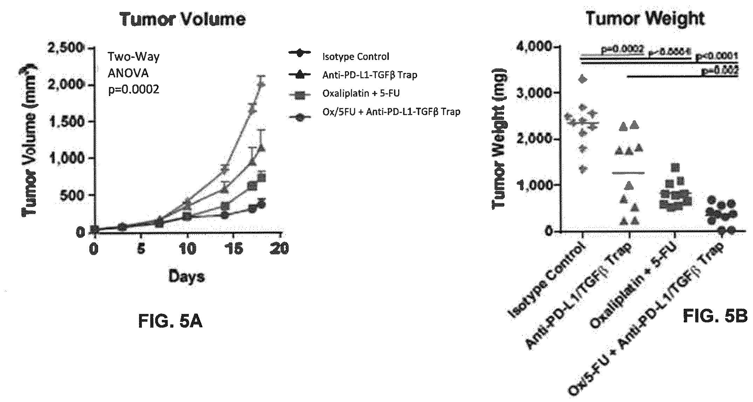

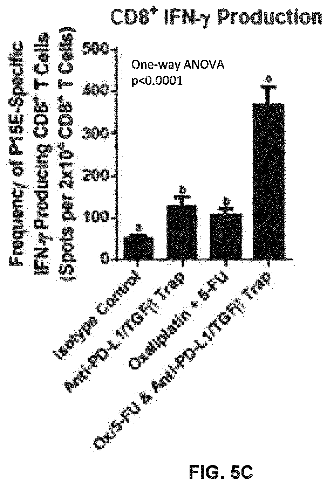

**FIG. 5A-5D** are a series of graphs showing that the Oxaliplatin/5-FU and anti-PD-L1/TGFβ trap combination enhances tumor growth inhibition and tumor-reactive CD8⁺T cell responses (B6.129S2-Ighm^{tm1Cgn}/J Mice; Study TI14-012). Fig. 5A and 4D. Tumor volume data was log transformed and a two-way, repeated measure ANOVA was performed. Fig. 5B. Tumor weight data was evaluated with one-way ANOVA. FIG. 5C. The frequency of IFN-γ producing, P15E-specific CD8⁺ T cells was quantified by ELISpot assay. ELISpot data was evaluated by one-way ANOVA. All ANOVA included Tukey's correction for multiple comparisons to measure statistical differences between treatment groups. p<0.05 was determined to be statistically significant.

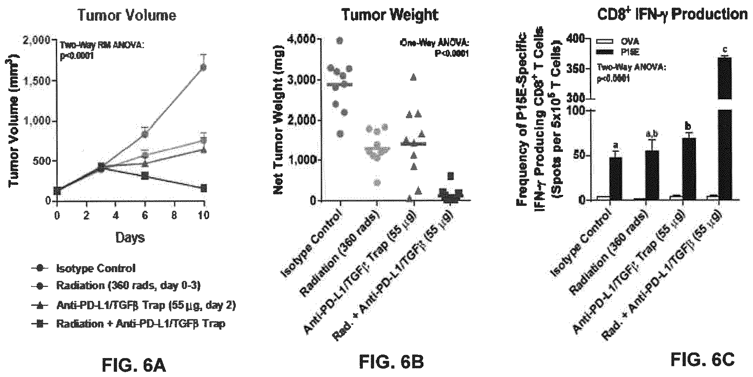

**FIG. 6A-6C** are a series of graphs showing that radiation and anti-PD-L1/TGFβ trap induces synergistic tumor growth inhibition and tumor-reactive CD8⁺ T Cell responses (TI13-109). FIG. 6A. Tumor volumes were measured twice per week and the average tumor volumes were presented as the mean ± standard error of the mean (SEM). FIG. 6B. Tumor weight data was determined on day 14. FIG. 6C. The frequency of IFN-γ producing, P15E-specific CD8⁺ T cells was quantified by ELISpot assay on day 14. The data of anti-PD-L1/TGFβ Trap at the dose level of 164 µg were similar to the data at the dose level of 55 µg, either as a monotherapy or the combination.
**FIG.7A-7C** are a series of graphs showing that radiation and anti-PD-L1/TGFβ trap induces synergistic tumor growth inhibition and tumor-Reactive CD8⁺T cell responses (repeat study) (TI14-013). FIG. 7A. Tumor volumes were measured twice per week and the average tumor volumes were presented as the mean ± standard error of the mean (SEM). FIG. 7B. Tumor weights were evaluated on day 14. FIG. 7C. The frequency of IFN-γ producing, P15E-specific CD8⁺T cells was quantified by ELISpot assay on day 14.
**FIG. 8A-8D** are a series of graphs showing that radiation and anti-PD-L1/TGFβ trap promotes tumor-infiltrating CD8⁺ T cells and NK cells (TI14-013). FIG. 8A. Tumor-infiltrating CD8⁺ TILS. FIG. 8B. Tumor-infiltrating NK1.1⁺ TILS. FIG. 8C. CD8⁺ TIL EOMES Expression. FIG. 8D. CD8⁺ TIL Degranulation.
**FIG. 9A** is a schematic diagram demonstrating the administration of radiation in a mouse carrying a primary and secondary tumor in order to test for an abscopal effect.
**FIG. 9B** is a line graph showing primary tumor volume in mice in the days since the start of treatment.
**FIG. 9C** is a line graph showing secondary tumor volume (mm³) in mice in the days since the start of treatment. (● = Isotype control 400 µg; ◆ = Anti-PDL1-TGFβ Trap µg; ■ = Radiation 500 rads; ▼ = Radiation + Anti-PDL1-TGFβ Trap).

### DETAILED DESCRIPTION

This disclosure relates generally to a combination therapy for the treatment of cancer, particularly to a combination of (i) a bifunctional molecule comprising a TGFβRII or fragment thereof capable of binding TGFβ and an antibody, or antigen binding fragment thereof, that binds to an immune checkpoint protein, such as Programmed Death Ligand 1 (PD-L1) and (ii) at least one additional anti-cancer therapeutic agent. Such anti-cancer therapeutic agents include, for example, radiation, chemotherapeutic agents, a biologic, and/or a vaccine. In certain disclosure herein, the combination therapy provides for a synergistic anti-cancer effect.

The combination therapy of the disclosure is particularly advantageous, since not only the anti-cancer effect is enhanced compared to the effect of each agent alone, but the dosage of the one or more agents in a combination therapy can be reduced as compared to monotherapy with each agent, while still achieving an overall anti-cancer effect. Due to the synergistic effect, the total amount of drugs administered to a patient can be advantageously reduced, thereby resulting in a decrease in side effects.

The combination therapy of the disclosure permits localized reduction in TGFβ in a tumor microenvironment by capturing the TGFβ using a soluble cytokine receptor (TGFβRII) tethered to an antibody moiety targeting a cellular immune checkpoint receptor found on the exterior surface of certain tumor cells or immune cells. An example of an antibody moiety of the disclosure is to an immune checkpoint protein is anti-PD-L1. This bifunctional molecule, sometimes referred to in this document as an "antibody-cytokine trap," is effective precisely because the anti-receptor antibody and cytokine trap are physically linked. The resulting advantage (over, for example, administration of the antibody and the receptor as separate molecules) is partly because cytokines function predominantly in the local environment through autocrine and paracrine functions. The antibody moiety directs the cytokine trap to the tumor microenvironment where it can be most effective, by neutralizing the local immunosuppressive autocrine or paracrine effects. Furthermore, in cases where the target of the antibody is internalized upon antibody binding, an effective mechanism for clearance of the cytokine/cytokine receptor complex is provided. Antibody-mediated target internalization has been shown for PD-L1. This is a distinct advantage over using an anti-TGFβ antibody because first, an anti-TGFβ antibody might not be completely neutralizing; and second, the antibody can act as a carrier extending the half-life of the cytokine, and antibody/cytokine complexes often act as a circulating sink that builds up and ultimately dissociates to release the cytokine back in circulation (Montero-Julian et al., Blood. 1995; 85:917-24). The use of a cytokine trap to neutralize the ligand can also be a better strategy than blockading the receptor with an antibody, as in the case of CSF-1. Because CSF-1 is cleared from the circulation by receptor-mediated endocytosis, an anti-CSF-1 receptor antibody blockade caused a significant increase in circulating CSF-1 concentration (Hume et al., Blood. 2012;119:1810-20)

As described below, treatment with the anti-PD-L1/TGFβ Trap, in combination with at least one additional anti-cancer therapeutic, elicits a synergistic anti-tumor effect due to the simultaneous blockade of the interaction between PD-L1 on tumor cells and PD-1 on immune cells, the neutralization of TGFβ in the tumor microenvironment, and the therapeutic effect of the anti-cancer agent. Without being bound by theory, this presumably is due to a synergistic effect obtained from simultaneous blocking the two major immune escape mechanisms, and in addition, the targeted depletion of the TGFβ in the tumor microenvironment by a single molecular entity, as well as the anti-tumor effect of the additional anti-cancer agent(s). This depletion is achieved by (1) anti-PD-L1 targeting of tumor cells; (2) binding of the TGFβ autocrine/paracrine in the tumor microenvironment by the TGFβ Trap; and (3) destruction of the bound TGFβ through the PD-L1 receptor-mediated endocytosis. The aforementioned mechanisms of action cannot be achieved by the combination therapy of the single agent anti-PD-L1, a TGFβ Trap and additional anti-cancer therapeutics. Furthermore, the TGFβRII fused to the C-terminus of Fc (fragment of crystallization of IgG) was several-fold more potent than the TGFpRII-Fc that places the TGFβRII at the N-terminus of Fc. The superb efficacy obtained with anti-PDL1/TGFβ Trap also allays some concerns that the TGFβRII does not trap TGFβ2. As pointed out by Yang et al., Trends Immunol. 2010; 31:220-227, although some tumor types do secrete TGFβ2 initially, as the tumor progresses, the TGFβ in the tumor microenvironment is predominantly secreted by myeloid-derived suppressor cells, which secrete TGFβ1. In addition to showing great promise as an effective immuno-oncology therapeutic, treatment with soluble TGFβRII can potentially reduce the cardiotoxicity concerns of TGFβ targeting therapies, especially the TGFβRI kinase inhibitors. This is because of the important roles TGFβ2 plays in embryonic development of the heart as well as in repair of myocardial damage after ischemia and reperfusion injury (Roberts et al., J Clin Invest. 1992; 90:2056-62).

### TGFβ as a cancer target

TGFβ had been a somewhat questionable target in cancer immunotherapy because of its paradoxical roles as the molecular Jekyll and Hyde of cancer (Bierie et al., Nat Rev Cancer. 2006; 6:506-20). Like some other cytokines, TGFβ activity is developmental stage and context dependent. Indeed TGFβ can act as either a tumor promoter or a tumor suppressor, affecting tumor initiation, progression and metastasis. The mechanisms underlying this dual role of TGFβ remain unclear (Yang et al., Trends Immunol. 2010; 31:220―227). Although it has been postulated that Smad-dependent signaling mediates the growth inhibition of TGFβ signaling, while the Smad independent pathways contribute to its tumor-promoting effect, there are also data showing that the Smad-dependent pathways are involved in tumor progression (Yang et al., Cancer Res. 2008; 68:9107-11).

Both the TGFβ ligand and the receptor have been studied intensively as therapeutic targets. There are three ligand isoforms, TGFβ1, 2 and 3, all of which exist as homodimers. There are also three TGFβ receptors (TGFβR), which are called TGFβR type I, II and III (López-Casillas et al., J Cell Biol. 1994; 124:557-68). TGFβRI is the signaling chain and cannot bind ligand. TGFβRII binds the ligand TGFβ1 and 3, but not TGFβ2, with high affinity. The TGFβRII/TGFβ complex recruits TGFβRI to form the signaling complex (Won et al., Cancer Res. 1999; 59:1273-7). TGFβRIII is a positive regulator of TGFβ binding to its signaling receptors and binds all 3 TGFβ isoforms with high affinity. On the cell surface, the TGFβ/TGFβRIII complex binds TGFβRII and then recruits TGFβRI, which displaces TGFβRIII to form the signaling complex.

Although the three different TGFβ isoforms all signal through the same receptor, they are known to have differential expression patterns and non-overlapping functions *in vivo.* The three different TGF-β isoform knockout mice have distinct phenotypes, indicating numerous non-compensated functions (Bujak et al., Cardiovasc Res. 2007; 74:184-95). While TGFβ1 null mice have hematopoiesis and vasculogenesis defects and TGFβ3 null mice display pulmonary development and defective palatogenesis, TGFβ2 null mice show various developmental abnormalities, the most prominent being multiple cardiac deformities (Bartram et al., Circulation. 2001; 103:2745-52; Yamagishi et al., Anat Rec. 2012; 295:257-67). Furthermore, TGFβ is implicated to play a major role in the repair of myocardial damage after ischemia and reperfusion injury. In an adult heart, cardiomyocytes secrete TGFβ, which acts as an autocrine to maintain the spontaneous beating rate. Importantly, 70-85% of the TGFβ secreted by cardiomyocytes is TGFβ2 (Roberts et al., J Clin Invest. 1992; 90:2056-62). In summary, given the predominant roles of TGFβ1 and TGFβ2 in the tumor microenvironment and cardiac physiology, respectively, a therapeutic agent that neutralizes TGFβ1 but not TGFβ2 could provide an optimal therapeutic index by minimizing the cardiotoxicity without compromising the anti-tumor activity. This is consistent with the findings by the present inventors, who observed a lack of toxicity, including cardiotoxicity, for anti-PD-L1/TGFβ Trap in monkeys.

Therapeutic approaches to neutralize TGFβ include using the extracellular domains of TGFβ receptors as soluble receptor traps and neutralizing antibodies. Of the receptor trap approach, soluble TGFβRIII may seem the obvious choice since it binds all the three TGFβ ligands. However, TGFβRIII, which occurs naturally as a 280-330 kD glucosaminoglycan (GAG)-glycoprotein, with extracellular domain of 762 amino acid residues, is a very complex protein for biotherapeutic development. The soluble TGFβRIII devoid of GAG could be produced in insect cells and shown to be a potent TGFβ neutralizing agent (Vilchis-Landeros et al, Biochem J 355:215, 2001). The two separate binding domains (the endoglin-related and the uromodulin-related) of TGFβRIII could be independently expressed, but they were shown to have affinities 20 to 100 times lower than that of the soluble TGFβRIII, and much diminished neutralizing activity (Mendoza et al., Biochemistry. 2009; 48:11755-65). On the other hand, the extracellular domain of TGFβRII is only 136 amino acid residues in length and can be produced as a glycosylated protein of 25-35 kD. The recombinant soluble TGFβRII was further shown to bind TGFβ1 with a K_{D} of 200 pM, which is fairly similar to the K_{D} of 50 pM for the full length TGFβRII on cells (Lin et al., J Biol Chem. 1995; 270:2747-54). Soluble TGFβRII-Fc was tested as an anti-cancer agent and was shown to inhibit established murine malignant mesothelioma growth in a tumor model (Suzuki et al., Clin Cancer Res. 2004; 10:5907-18). Since TGFβRII does not bind TGFβ2, and TGFβRIII binds TGFβ1 and 3 with lower affinity than TGFβRII, a fusion protein of the endoglin domain of TGFβRIII and extracellular domain of TGFβRII was produced in bacteria and was shown to inhibit the signaling of TGFβ1 and 2 in cell based assays more effectively than either TGFβRII or RIII (Verona et al., Protein Eng Des Sel. 2008; 21:463-73). Despite some encouraging anti-tumor activities in tumor models, to our knowledge no TGFβ receptor trap recombinant proteins have been tested in the clinic.

Still another approach to neutralize all three isoforms of the TGFβ ligands is to screen for a pan-neutralizing anti-TGFβ antibody, or an anti-receptor antibody that blocks the receptor from binding to TGFβ1, 2 and 3. GC1008, a human antibody specific for all isoforms of TGFβ, was in a Phase I/II study in patients with advanced malignant melanoma or renal cell carcinoma (Morris et al., J Clin Oncol 2008; 26:9028 (Meeting abstract)). Although the treatment was found to be safe and well tolerated, only limited clinical efficacy was observed, and hence it was difficult to interpret the importance of anti-TGFβ therapy without further characterization of the immunological effects (Flavell et al., Nat Rev Immunol. 2010; 10:554-67). There were also TGFβ-isoform-specific antibodies tested in the clinic. Metelimumab, an antibody specific for TGFβ1 was tested in Phase 2 clinical trial as a treatment to prevent excessive post-operative scarring for glaucoma surgery; and Lerdelimumab, an antibody specific for TGFβ2, was found to be safe but ineffective at improving scarring after eye surgery in a Phase 3 study (Khaw et al., Ophthalmology 2007; 114:1822-1830). Anti-TGFβRII antibodies that block the receptor from binding to all three TGFβ isoforms, such as the anti-human TGFβRII antibody TR1 and anti-mouse TGFβRII antibody MT1, have also shown some therapeutic efficacy against primary tumor growth and metastasis in mouse models (Zhong et al., Clin Cancer Res. 2010; 16:1191-205). To date, the vast majority of the studies on TGFβ targeted anticancer treatment, including small molecule inhibitors of TGFβ signaling that often are quite toxic, are mostly in the preclinical stage and the anti-tumor efficacy obtained has been limited (Calone et al., Exp Oncol. 2012; 34:9-16; Connolly et al., Int J Biol Sci. 2012; 8:964-78).

The antibody-TGFβ trap of the disclosure, for use in the combination therapy of the disclosure, is a bifunctional protein containing at a least portion of a human TGFβ Receptor II (TGFβRII) that is capable of binding TGFβ. In some disclosure, the TGFβ trap polypeptide is a soluble portion of the human TGFβ Receptor Type 2 Isoform A (SEQ ID NO: 8) that is capable of binding TGFβ. In a further disclosure, TGFβ trap polypeptide contains at least amino acids 73-184 of SEQ ID NO:8. In yet a further disclosure,, the TGFβ trap polypeptide contains amino acids 24-184 of SEQ ID NO:8. In another disclosure,t, the TGFβ trap polypeptide is a soluble portion of the human TGFβ Receptor Type 2 Isoform B (SEQ ID NO: 9) that is capable of binding TGFβ. In a further disclosure,, TGFβ trap polypeptide contains at least amino acids 48-159 of SEQ ID NO:9. In yet a further disclosure, the TGFβ trap polypeptide contains amino acids 24-159 of SEQ ID NO:9. In yet a further disclosure, the TGFβ trap polypeptide contains amino acids 24-105 of SEQ ID NO:9.

### Immune Checkpoint Dis-inhibition

The approach of targeting T cell inhibition checkpoints for dis-inhibition with therapeutic antibodies is an area of intense investigation (for a review, see Pardoll, Nat Rev Cancer. 2012; 12:253-264). In one approach, the antibody moiety or antigen binding fragment thereof targets T cell inhibition checkpoint receptor proteins on the T cell, such as, for example: CTLA-4, PD-1, BTLA, LAG-3, TIM-3, and LAIR1. In another approach, the antibody moiety targets the counter-receptors on antigen presenting cells and tumor cells (which co-opt some of these counter-receptors for their own immune evasion), such as, for example: PD-L1 (B7-H1), B7-DC, HVEM, TIM-4, B7-H3, or B7-H4.

The disclosure contemplates the use of antibody TGFβ traps that target, through their antibody moiety or antigen binding fragment thereof, T cell inhibition checkpoints for dis-inhibition. To that end the present inventors have tested the anti-tumor efficacy of combining a TGFβ trap with antibodies targeting various T cell inhibition checkpoint receptor proteins, such as anti-PD-1, anti-PD-L1, anti-TIM-3 and anti-LAG3. The present inventors found that combining a TGFβ trap with an anti-PD-L1 antibody exhibited remarkable anti-tumor activity beyond what was observed with the monotherapies. In contrast, none of the other combinations with antibodies to the targets listed above showed any superior efficacy. In particular, one may have expected that a combination treatment of a TGFβ trap with an anti-PD-1 antibody would demonstrate similar activity to the one observed with anti-PD-L1, as PD-1 / PD-L1 are cognate receptors that bind to each other to effect the immune checkpoint inhibition. However, this is not what the present inventors have found.

### Anti-PD-L1 Antibodies

The disclosure can include the use of any anti-PD-L1 antibody, or antigen-binding fragment thereof, described in the art. Anti-PD-L1 antibodies are commercially available, for example, the 29E2A3 antibody (Biolegend, Cat. No. 329701). Antibodies can be monoclonal, chimeric, humanized, or human. Antibody fragments include Fab, F(ab')2, scFv and Fv fragments, which are described in further detail below.

Exemplary antibodies are described in PCT Publication WO 2013/079174. These antibodies can include a heavy chain variable region polypeptide including an HVR-H1, HVR-H2, and HVR-H3 sequence, where:
(a) the HVR-H1 sequence is X₁YX₂MX₃;
(b) the HVR-H2 sequence is SIYPSGGX₄TFYADX₅VKG;
(c) the HVR-H3 sequence is IKLGTVTTVX₆Y;
   further where: X₁ is K, R, T, Q, G, A, W, M, I, or S; X₂ is V, R, K, L, M, or I; X₃ is H, T, N, Q, A, V, Y, W, F, or M; X₄ is F or I; X₅ is S or T; X₆ is E or D.

In some disclosure, X₁ is M, I, or S; X₂ is R, K, L, M, or I; X₃ is F or M; X₄ is F or I; X₅ is S or T; X₆ is E or D.

In another disclosure, X₁ is M, I, or S; X₂ is L, M, or I; X₃ is F or M; X₄ is I; X₅ is S or T; X₆ is D.

In still another disclosure, X₁ is S; X₂ is I; X₃ is M; X₄ is I; X₅ is T; X₆ is D.

In other disclosure,, the polypeptide further includes variable region heavy chain framework sequences juxtaposed between the HVRs according to the formula: (HC-FR1)-(HVR-H1)-(HC-FR2)-(HVR-H2)-(HC-FR3)-(HVR-H3)-(HC-FR4).

In yet other disclosure, the framework sequences are derived from human consensus framework sequences or human germline framework sequences.

In a still further aspect, at least one of the framework sequences is the following:
HC-FR1 is EVQLLESGGGLVQPGGSLRLSCAASGFTFS;
HC-FR2 is WVRQAPGKGLEWVS;
HC-FR3 is RFTISRDNSKNTLYLQMNSLRAEDTAVYYCAR;
HC-FR4 is WGQGTLVTVSS.

In a still further disclosure, the heavy chain polypeptide is further combined with a variable region light chain including an HVR-L1, HVR-L2, and HVR-L3, where:
(a) the HVR-L1 sequence is TGTX₇X₈DVGX₉YNYVS;
(b) the HVR-L2 sequence is X₁₀VX₁₁X₁₂RPS;
(c) the HVR-L3 sequence is SSX₁₃TX₁₄X₁₅X₁₆X₁₇RV;
   further where: X₇ is N or S; X₈ is T, R, or S; X₉ is A or G; X₁₀ is E or D; X₁₁ is I, N or S; X₁₂ is D, HorN; X₁₃ is F or Y; X₁₄ is N or S; X₁₅ is R, T or S; X₁₆ is G or S; X₁₇ is I or T.

In other disclosure, X₇ is N or S; X₈ is T, R, or S; X₉ is A or G; X₁₀ is E or D; X₁₁ is N or S; X₁₂ is N; X₁₃ is F or Y; X₁₄ is S; X₁₅ is S; X₁₆ is G or S; X₁₇ is T.

In still other disclosure, X₇ is S; X₈ is S; X₉ is G; X₁₀ is D; X₁₁ is S; X₁₂ is N; X₁₃ is Y; X₁₄ is S; X₁₅ is S; X₁₆ is S; X₁₇ is T.

In a still further disclosure,, the light chain further includes variable region light chain framework sequences juxtaposed between the HVRs according to the formula: (LC-FR1)-(HVR-L1)-(LC-FR2)-(HVR-L2)-(LC-FR3)-(HVR-L3)-(LC-FR4).

In a still further disclosure,, the light chain framework sequences are derived from human consensus framework sequences or human germline framework sequences.

In a still further disclosure, the light chain framework sequences are lambda light chain sequences.

In a still further disclosure, at least one of the framework sequence is the following:
LC-FR1 is QSALTQPASVSGSPGQSITISC;
LC-FR2 is WYQQHPGKAPKLMIY;
LC-FR3 is GVSNRFSGSKSGNTASLTISGLQAEDEADYYC;
LC-FR4 is FGTGTKVTVL.

Other disclosure herein provides an anti-PD-L1 antibody or antigen binding fragment including a heavy chain and a light chain variable region sequence, where:
(a) the heavy chain includes an HVR-H1, HVR-H2, and HVR-H3, wherein further: (i) the HVR-H1 sequence is X₁YX₂MX₃; (ii) the HVR-H2 sequence is SIYPSGGX₄TFYADX₅VKG; (iii) the HVR-H3 sequence is IKLGTVTTVX₆Y, and;
(b) the light chain includes an HVR-L1, HVR-L2, and HVR-L3, wherein further: (iv) the HVR-L1 sequence is TGTX₇X₈DVGX₉YNYVS; (v) the HVR-L2 sequence is X₁₀VX₁₁X₁₂RPS; (vi) the HVR-L3 sequence is SSX₁₃TX₁₄X₁₅X₁₆X₁₇RV; wherein: X₁ is K, R, T, Q, G, A, W, M, I, or S; X₂ is V, R, K, L, M, or I; X₃ is H, T, N, Q, A, V, Y, W, F, or M; X₄ is F or I; X₅ is S or T; X₆ is E or D; X₇ is N or S; X₈ is T, R, or S; X₉ is A or G; X₁₀ is E or D; X₁₁ is I, N, or S; X₁₂ is D, H, or N; X₁₃ is F or Y; X₁₄ is N or S; X₁₅ is R, T, or S; X₁₆ is G or S; X₁₇ is I or T.

In some disclosure herein, X₁ is M, I, or S; X₂ is R, K, L, M, or I; X₃ is F or M; X₄ is F or I; X₅ is S or T; X₆ is E or D; X₇ is N or S; X₈ is T, R, or S; X₉ is A or G; X₁₀ is E or D; X₁₁is N or S; X₁₂ is N; X₁₃ is F or Y; X₁₄ is S; X₁₅ is S; X₁₆ is G or S; X₁₇ is T.

In other disclosure, X₁ is M, I, or S; X₂ is L, M, or I; X₃ is F or M; X₄ is I; X₅ is S or T; X₆ is D; X₇ is N or S; X₈ is T, R, or S; X₉ is A or G; X₁₀ is E or D; X₁₁ is N or S; X₁₂ is N; X₁₃ is F or Y; X₁₄ is S; X₁₅ is S; X₁₆ is G or S; X₁₇ is T.

In still other disclosure, X₁ is S; X₂ is I; X₃ is M; X₄ is I; X₅ is T; X₆ is D; X₇ is S; X₈ is S; X₉ is G; X₁₀ is D; X₁₁ is S; X₁₂ is N; X₁₃ is Y; X₁₄ is S; X₁₅ is S; X₁₆ is S; X₁₇ is T.

In a further disclosure, the heavy chain variable region includes one or more framework sequences juxtaposed between the HVRs as: (HC-FR1)-(HVR-H1)-(HC-FR2)-(HVR-H2)-(HC-FR3)-(HVR-H3)-(HC-FR4), and the light chain variable regions include one or more framework sequences juxtaposed between the HVRs as: (LC-FR1 MHVR-L1 )-(LC-FR2)-(HVR-L2)-(LC-FR3)-(HVR-L3)-(LC-FR4).

In a still further disclosure, the framework sequences are derived from human consensus framework sequences or human germline sequences.

In a still further disclosure, one or more of the heavy chain framework sequences is the following:
HC-FR1 is EVQLLESGGGLVQPGGSLRLSCAASGFTFS;
HC-FR2 is WVRQAPGKGLEWVS;
HC-FR3 is RFTISRDNSKNTLYLQMNSLRAEDTAVYYCAR;
HC-FR4 is WGQGTLVTVSS.

In a still further disclosure, the light chain framework sequences are lambda light chain sequences.

In a still further disclosure, one or more of the light chain framework sequences is the following:
LC-FR1 is QSALTQPASVSGSPGQSITISC;
LC-FR2 is WYQQHPGKAPKLMIY;
LC-FR3 is GVSNRFSGSKSGNTASLTISGLQAEDEADYYC;
LC-FR4 is FGTGTKVTVL.

In a still further disclosure, the heavy chain variable region polypeptide, antibody, or antibody fragment further includes at least a C_{H}1 domain.

In a more specific disclosure, the heavy chain variable region polypeptide, antibody, or antibody fragment further includes a C_{H}1, a C_{H}2, and a C_{H}3 domain.

In a still further disclosure, the variable region light chain, antibody, or antibody fragment further includes a C_{L} domain.

In a still further disclosure, the antibody further includes a C_{H}1, a C_{H}2, a C_{H}3, and a C_{L} domain.

In a still further specific disclosure, the antibody further includes a human or murine constant region.

In a still further disclosure, the human constant region is selected from the group consisting of IgG1, IgG2, IgG2, IgG3, and IgG4.

In a still further specific disclosure, the human or murine constant region is lgG1.

Other disclosure herein features an anti-PD-L1 antibody including a heavy chain and a light chain variable region sequence, where:
(a) the heavy chain includes an HVR-H1, an HVR-H2, and an HVR-H3, having at least 80% overall sequence identity to SYIMM, SIYPSGGITFYADTVKG, and IKLGTVTTVDY, respectively, and
(b) the light chain includes an HVR-L1, an HVR-L2, and an HVR-L3, having at least 80% overall sequence identity to TGTSSDVGGYNYVS, DVSNRPS, and SSYTSSSTRV, respectively.

In a specific disclosure, the sequence identity is 81%, 82%, 83%, 84%, 85%, 86%, 87%, 88%, 89%, 90%, 91%, 92%, 93%, 94%, 95%, 96%, 97%, 98%, 99%, or 100%.

Other disclosure features an anti-PD-L1 antibody including a heavy chain and a light chain variable region sequence, where:
(a) the heavy chain includes an HVR-H1, an HVR-H2, and an HVR-H3, having at least 80% overall sequence identity to MYMMM, SIYPSGGITFYADSVKG, and IKLGTVTTVDY, respectively, and
(b) the light chain includes an HVR-L1, an HVR-L2, and an HVR-L3, having at least 80% overall sequence identity to TGTSSDVGAYNYVS, DVSNRPS, and SSYTSSSTRV, respectively.

In a specific disclosure, the sequence identity is 81%, 82%, 83%, 84%, 85%, 86%, 87%, 88%, 89%, 90%, 91%, 92%, 93%, 94%, 95%, 96%, 97%, 98%, 99%, or 100%.

In a still further disclosure, in the antibody or antibody fragment according to the disclosure, as compared to the sequences of HVR-H1, HVR-H2, and HVR-H3, at least those amino acids remain unchanged that are highlighted by underlining as follows:
(a) in HVR-H1 SYIMM,
(b) in HVR-H2 SIYPSGGITFYADTVKG,
(c) in HVR-H3 IKLGTVTTVDY;
and further where, as compared to the sequences of HVR-L1, HVR-L2, and HVR-L3 at least those amino acids remain unchanged that are highlighted by underlining as follows:
(a) HVR-L1 TGTSSDVGGYNYVS
(b) HVR-L2 DVSNRPS
(c) HVR-L3 SSYTSSSTRV.

In another disclosuret, the heavy chain variable region includes one or more framework sequences juxtaposed between the HVRs as: (HC-FR1)-(HVR-H1)-(HC-FR2)-(HVR-H2)-(HC-FR3)-(HVR-H3)-(HC-FR4), and the light chain variable regions include one or more framework sequences juxtaposed between the HVRs as: (LC-FR1)-(HVR-L1)-(LC-FR2)-(HVR-L2)-(LC-FR3)-(HVR-L3)-(LC-FR4).

In yet another disclosure, the framework sequences are derived from human germline sequences.

In a still further disclosure, one or more of the heavy chain framework sequences is the following:
HC-FR1 is EVQLLESGGGLVQPGGSLRLSCAASGFTFS;
HC-FR2 is WVRQAPGKGLEWVS;
HC-FR3 is RFTISRDNSKNTLYLQMNSLRAEDTAVYYCAR;
HC-FR4 is WGQGTLVTVSS.

In a still further disclosure, the light chain framework sequences are derived from a lambda light chain sequence.

In a still further disclosure, one or more of the light chain framework sequences is the following:
LC-FR1 is QSALTQPASVSGSPGQSITISC;
LC-FR2 is WYQQHPGKAPKLMIY;
LC-FR3 is GVSNRFSGSKSGNTASLTISGLQAEDEADYYC;
LC-FR4 is FGTGTKVTVL.

In a still further specific disclosure, the antibody further includes a human or murine constant region.

In a still further disclosure, the human constant region is selected from the group consisting of IgG1, IgG2, IgG2, IgG3, and IgG4.

Still further disclosure features an anti-PD-L1 antibody including a heavy chain and a light chain variable region sequence, where:
(a) the heavy chain sequence has at least 85% sequence identity to the heavy chain sequence:
   EVQLLESGGGLVQPGGSLRLSCAASGFTFSSYIMMVWRQAPGKGLEWVSSIYPSGGITF YADWKGRFTISRDNSKNTLYLQMNSLRAEDTAVYYCARIKLGTVTTVDYWGQGTLV TVSS, and
(b) the light chain sequence has at least 85% sequence identity to the light chain sequence:

QSALTQPASVSGSPGQSITISCTGTSSDVGGYNYVSWYQQHPGKAPKLMIYDVSN
RPSGVSNRFSGSKSGNTASLTISGLQAEDEADYYCSSYTSSSTRVFGTGTKVTVL.

In a specific disclosure, the sequence identity is 86%, 87%, 88%, 89%, 90%, 91%, 92%, 93%, 94%, 95%, 96%, 97%, 98%, 99%, or 100%.

Still further disclosure provides for an anti-PD-L1 antibody including a heavy chain and a light chain variable region sequence, where:
(a) the heavy chain sequence has at least 85% sequence identity to the heavy chain sequence:
   EVQLLESGGGLVQPGGSLRLSCAASGFTFSMYMMMWVRQAPGKGLEVWSSIYPSGGI TFYADSVKGRFTISRDNSKNTLYLQMNSLRAEDTAIYYCARIKLGTVTTVDYWG QGTLVTVSS, and
(b) the light chain sequence has at least 85% sequence identity to the light chain sequence:

QSALTQPASVSGSPGQSITISCTGTSSDVGAYNYVSWYQQHPGKAPKLMIYDVSNR
PSGVSNRFSGSKSGNTASLTISGLQAEDEADYYCSSYTSSSTRVFGTGTKVTVL.

In a specificdisclosure, the sequence identity is 86%, 87%, 88%, 89%, 90%, 91%, 92%, 93%, 94%, 95%, 96%, 97%, 98%, 99% or 100%.In another embodiment the antibody binds to human, mouse, or cynomolgus monkey PD-L1. In a specific aspect the antibody is capable of blocking the interaction between human, mouse, or cynomolgus monkey PD-L1 and the respective human, mouse, or cynomolgus monkey PD-1 receptors.

In other disclosure, the antibody binds to human PD-L1 with a K_{D} of 5×10⁻⁹ M or less, preferably with a K_{D} of 2×10⁻⁹ M or less, and even more preferred with a K_{D} of 1×10⁻⁹ M or less.

Other disclosure relates to an anti-PD-L1 antibody or antigen binding fragment thereof which binds to a functional epitope including residues Y56 and D61 of human PD-L1.

In a specific disclosure, the functional epitope further includes E58, E60, Q66, R113, and M115 of human PD-L1.

In a more specific disclosure, the antibody binds to a conformational epitope, including residues 54-66 and 112-122 of human PD-L1.

Further disclosure is related to the use of an anti-PD-L1 antibody, or antigen binding fragment thereof, which cross-competes for binding to PD-L1 with an antibody according to the disclosure as described herein.

Still further disclosure features proteins and polypeptides including any of the above described anti-PD-L1 antibodies in combination with at least one pharmaceutically acceptable carrier for use in the combination therapy of the disclosure.

Still further disclosure features the use of an isolated nucleic acid encoding a polypeptide, or light chain or a heavy chain variable region sequence of an anti-PD-L1 antibody, or antigen binding fragment thereof, as described herein. Still further disclosure provides for an isolated nucleic acid encoding a light chain or a heavy chain variable region sequence of an anti-PD-L1 antibody, wherein:
(a) the heavy chain includes an HVR-H1, an HVR-H2, and an HVR-H3 sequence having at least 80% sequence identity to SYIMM, SIYPSGGITFYADTVKG, and IKLGTVTTVDY, respectively, or
(b) the light chain includes an HVR-L1, an HVR-L2, and an HVR-L3 sequence having at least 80% sequence identity to TGTSSDVGGYNYVS, DVSNRPS, and SSYTSSSTRV, respectively.

In a specific disclosure, the sequence identity is 81%, 82%, 83%, 84%, 85%, 86%, 87%, 88%, 89%, 90%, 91%, 92%, 93%, 94%, 95%, 96%, 97%, 98%, 99%, or 100%.

In a further disclosure, the nucleic acid sequence for the heavy chain is: 
atggagttgc ctgttaggct gttggtgctg atgttctgga ttcctgctag ctccagcgag 60
gtgcagctgc tggaatccgg cggaggactg gtgcagcctg gcggctccct gagactgtct 120
tgcgccgcct ccggcttcac cttctccagc tacatcatga tgtgggtgcg acaggcccct 180
ggcaagggcc tggaatgggt gtcctccatc tacccctccg gcggcatcac cttctacgcc 240
gacaccgtga agggccggtt caccatctcc cgggacaact ccaagaacac cctgtacctg 300
cagatgaact ccctgcgggc cgaggacacc gccgtgtact actgcgcccg gatcaagctg 360
ggcaccgtga ccaccgtgga ctactggggc cagggcaccc tggtgacagt gtcctccgcc 420
tccaccaagg gcccatcggt cttccccctg gcaccctcct ccaagagcac ctctgggggc 480
acagcggccc tgggctgcct ggtcaaggac tacttccccg aaccggtgac ggtgtcgtgg 540
aactcaggcg ccctgaccag cggcgtgcac accttcccgg ctgtcctaca gtcctcagga 600
ctctactccc tcagcagcgt ggtgaccgtg ccctccagca gcttgggcac ccagacctac 660
atctgcaacg tgaatcacaa gcccagcaac accaaggtgg acaagaaagt tgagcccaaa 720
tcttgtgaca aaactcacac atgcccaccg tgcccagcac ctgaactcct ggggggaccg 780
tcagtcttcc tcttcccccc aaaacccaag gacaccctca tgatctcccg gacccctgag 840
gtcacatgcg tggtggtgga cgtgagccac gaagaccctg aggtcaagtt caactggtac 900
gtggacggcg tggaggtgca taatgccaag acaaagccgc gggaggagca gtacaacagc 960
acgtaccgtg tggtcagcgt cctcaccgtc ctgcaccagg actggctgaa tggcaaggag 1020
tacaagtgca aggtctccaa caaagccctc ccagccccca tcgagaaaac catctccaaa 1080
gccaaagggc agccccgaga accacaggtg tacaccctgc ccccatcacg ggatgagctg 1140
accaagaacc aggtcagcct gacctgcctg gtcaaaggct tctatcccag cgacatcgcc 1200
gtggagtggg agagcaatgg gcagccggag aacaactaca agaccacgcc tcccgtgctg 1260
gactccgacg gctccttctt cctctatagc aagctcaccg tggacaagag caggtggcag 1320
caggggaacg tcttctcatg ctccgtgatg catgaggctc tgcacaacca ctacacgcag 1380
aagagcctct ccctgtcccc gggtaaa 1407
 and the nucleic acid sequence for the light chain is:

atggagttgc ctgttaggct gttggtgctg atgttctgga ttcctgcttc cttaagccag 60

tccgccctga cccagcctgc ctccgtgtct ggctcccctg gccagtccat caccatcagc 120

tgcaccggca cctccagcga cgtgggcggc tacaactacg tgtcctggta tcagcagcac 180

cccggcaagg cccccaagct gatgatctac gacgtgtcca accggccctc cggcgtgtcc 240

aacagattct ccggctccaa gtccggcaac accgcctccc tgaccatcag cggactgcag 300

gcagaggacg aggccgacta ctactgctcc tcctacacct cctccagcac cagagtgttc 360

ggcaccggca caaaagtgac cgtgctgggc cagcccaagg ccaacccaac cgtgacactg 420

ttccccccat cctccgagga actgcaggcc aacaaggcca ccctggtctg cctgatctca 480

gatttctatc caggcgccgt gaccgtggcc tggaaggctg atggctcccc agtgaaggcc 540

ggcgtggaaa ccaccaagcc ctccaagcag tccaacaaca aatacgccgc ctcctcctac 600

ctgtccctga cccccgagca gtggaagtcc caccggtcct acagctgcca ggtcacacac 660

gagggctcca ccgtggaaaa gaccgtcgcc cccaccgagt gctca 705

Further exemplary anti-PD-L1 antibodies that can be used in an anti-PD-L1/TGFβ Trap are described in US patent application publication US 2010/0203056. In some disclosure, the antibody moiety is YW243.55S70. In other disclosure, the antibody moiety is MPDL3280A.

A further disclosure features the use of an anti-PD-L 1 antibody moiety including a heavy chain and a light chain variable region sequence, where:
(a) the heavy chain sequence has at least 85% sequence identity to the heavy chain sequence:
   EVQLVESGGGLVQPGGSLRLSCAASGFTFSDSWIHWVRQAPGKGLEWVAWISPYGGSTYY ADSVKGRFTISADTSKNTAYLQMNSLRAEDTAVYYCARRHWPGGFDYWGQGTLVTVSS (SEQ ID NO: 12), and
(b) the light chain sequence has at least 85% sequence identity to the light chain sequence:

DIQMTQSPSSLSASVGDRVTITCRASQDVSTAVAWYQQKPGKAPKLLIYSASFLYSGVPSRFS
GSGSGTDFTLTISSLQPEDFATYYCQQYLYHPATFGQGTKVEIKR (SEQ ID NO:13).

In a specific disclosure, the sequence identity is 86%, 87%, 88%, 89%, 90%, 91%, 92%, 93%, 94%, 95%, 96%, 97%, 98%, or 99%.

Further disclosure features the use of an anti-PD-L1 antibody moiety including a heavy chain and a light chain variable region sequence, where:
(a) the heavy chain variable region sequence is:
   EVQLVESGGGLVQPGGSLRLSCAASGFTFSDSWIHWVRQAPGKGLEWVAWISPYGGSTYY ADSVKGRFTISADTSKNTAYLQMNSLRAEDTAVYYCARRHWPGGFDYWGQGTLVTVSS (SEQ ID NO: 12), and
(b) the light chain variable region sequence is:

DIQMTQSPSSLSASVGDRVTITCRASQDVSTAVAWYQQKPGKAPKLLIYSASFLYSGVPSRFS
GSGSGTDFTLTISSLQPEDFATYYCQQYLYHPATFGQGTKVEIKR (SEQ ID NO:13).

Further disclosure features an anti-PD-L1 antibody moiety including a heavy chain and a light chain variable region sequence, where:
(a) the heavy chain variable region sequence is:
   EVQLVESGGGLVQPGGSLRLSCAASGFTFSDSWIHWVRQAPGKGLEWVAWISPYGGSTYYADS VKGRFTISADTSKNTAYLQMNSLRAEDTAVYYCARRHWPGGFDYWGQGTLVTVSA (SEQID NO:14), and
(b) the light chain variable region sequence is:

DIQMTQSPSSLSASVGDRVTITCRASQDVSTAVAWYQQKPGKAPKLLIYSASFLYSGVPSRFS
GSGSGTDFTLTISSLQPEDFATYYCQQYLYHPATFGQGTKVEIKR (SEQ ID NO:13).

Yet further exemplary anti-PD-L1 antibodies that can be used in an anti-PD-L1/TGFβ Trap are described in US patent publication US 7,943,743.

In some disclosure, the anti-PD-L1 antibody is MDX-1105.

In yet further disclosure, the anti-PD-L1 antibody is MEDI-4736.

### Constant region

The proteins and peptides for use in the combination therapy of the disclosure can include a constant region of an immunoglobulin or a fragment, analog, variant, mutant, or derivative of the constant region. In preferred disclosure herein, the constant region is derived from a human immunoglobulin heavy chain, for example, IgG1, IgG2, IgG3, IgG4, or other classes. In disclosure herein, the constant region includes a CH2 domain. In other disclosure herein the constant region includes CH2 and CH3 domains or includes hinge-CH2-CH3. Alternatively, the constant region can include all or a portion of the hinge region, the CH2 domain and/or the CH3 domain.

In disclosure herein, the constant region contains a mutation that reduces affinity for an Fc receptor or reduces Fc effector function. For example, the constant region can contain a mutation that eliminates the glycosylation site within the constant region of an IgG heavy chain. In some disclosure herein, the constant region contains mutations, deletions, or insertions at an amino acid position corresponding to Leu234, Leu235, Gly236, Gly237, Asn297, or Pro331 of IgG1 (amino acids are numbered according to EU nomenclature). In particular disclosure herein, the constant region contains a mutation at an amino acid position corresponding to Asn297 of IgG1. In alternative disclosure herein, the constant region contains mutations, deletions, or insertions at an amino acid position corresponding to Leu281, Leu282, Gly283, Gly284, Asn344, or Pro378 of IgG1.

In some disclosure herein, the constant region contains a CH2 domain derived from a human IgG2 or IgG4 heavy chain. Preferably, the CH2 domain contains a mutation that eliminates the glycosylation site within the CH2 domain. In disclosure hereint, the mutation alters the asparagine within the Gln-Phe-Asn-Ser (SEQ ID NO: 15) amino acid sequence within the CH2 domain of the IgG2 or IgG4 heavy chain. Preferably, the mutation changes the asparagine to a glutamine. Alternatively, the mutation alters both the phenylalanine and the asparagine within the Gln-Phe-Asn-Ser (SEQ ID NO: 15) amino acid sequence. In disclosure herein, the Gln-Phe-Asn-Ser (SEQ ID NO: 15) amino acid sequence is replaced with a Gln-Ala-Gln-Ser (SEQ ID NO: 16) amino acid sequence. The asparagine within the Gln-Phe-Asn-Ser (SEQ ID NO: 15) amino acid sequence corresponds to Asn297 of IgG1.

In other disclosure, the constant region includes a CH2 domain and at least a portion of a hinge region. The hinge region can be derived from an immunoglobulin heavy chain, e.g., IgG1, IgG2, IgG3, IgG4, or other classes. Preferably, the hinge region is derived from human IgG1, IgG2, IgG3, IgG4, or other suitable classes. More preferably the hinge region is derived from a human IgG1 heavy chain. In some disclosure, the cysteine in the Pro-Lys-Ser-Cys-Asp-Lys (SEQ ID NO: 17) amino acid sequence of the IgG1 hinge region is altered. In some disclosure the Pro-Lys-Ser-Cys-Asp-Lys (SEQ ID NO: 17) amino acid sequence is replaced with a Pro-Lys-Ser-Ser-Asp-Lys (SEQ ID NO: 18) amino acid sequence. In some disclosure, the constant region includes a CH2 domain derived from a first antibody isotype and a hinge region derived from a second antibody isotype. In some disclosure, the CH2 domain is derived from a human IgG2 or IgG4 heavy chain, while the hinge region is derived from an altered human IgG1 heavy chain.

The alteration of amino acids near the junction of the Fc portion and the non-Fc portion can dramatically increase the serum half-life of the Fc fusion protein (PCT publication WO 01/58957). Accordingly, the junction region of a protein or polypeptide of the present disclosure can contain alterations that, relative to the naturally-occurring sequences of an immunoglobulin heavy chain and erythropoietin, preferably lie within about 10 amino acids of the junction point. These amino acid changes can cause an increase in hydrophobicity. In some disclosure, the constant region is derived from an IgG sequence in which the C-terminal lysine residue is replaced. Preferably, the C-terminal lysine of an IgG sequence is replaced with a non-lysine amino acid, such as alanine or leucine, to further increase serum half-life. In other disclosure, the constant region is derived from an IgG sequence in which the Leu-Ser-Leu-Ser (SEQ ID NO: 19) amino acid sequence near the C-terminus of the constant region is altered to eliminate potential junctional T-cell epitopes. For example, in some disclosure, the Leu-Ser-Leu-Ser amino acid sequence is replaced with an Ala-Thr-Ala-Thr (SEQ ID NO: 20) amino acid sequence. In other disclosure herein, the amino acids within the Leu-Ser-Leu-Ser (SEQ ID NO: 19) segment are replaced with other amino acids such as glycine or proline. Detailed methods of generating amino acid substitutions of the Leu-Ser-Leu-Ser (SEQ ID NO: 19) segment near the C-terminus of an IgG1, IgG2, IgG3, IgG4, or other immunoglobulin class molecule have been described in U.S. Patent Publication No. 2003/0166877.

Suitable hinge regions for the present disclosure can be derived from IgG1, IgG2, IgG3, IgG4, and other immunoglobulin classes. The IgG1 hinge region has three cysteines, two of which are involved in disulfide bonds between the two heavy chains of the immunoglobulin. These same cysteines permit efficient and consistent disulfide bonding formation between Fc portions. Therefore, a preferred hinge region of the present disclosure is derived from IgG1, more preferably from human IgG1. In some disclosure herein, the first cysteine within the human IgG1 hinge region is mutated to another amino acid, preferably serine. The IgG2 isotype hinge region has four disulfide bonds that tend to promote oligomerization and possibly incorrect disulfide bonding during secretion in recombinant systems. A suitable hinge region can be derived from an IgG2 hinge; the first two cysteines are each preferably mutated to another amino acid. The hinge region of IgG4 is known to form interchain disulfide bonds inefficiently. However, a suitable hinge region for the present disclosure can be derived from the IgG4 hinge region, preferably containing a mutation that enhances correct formation of disulfide bonds between heavy chain-derived moieties (Angal S, et al. (1993) Mol. Immunol., 30:105-8).

In accordance with the present disclosure, the constant region can contain CH2 and/or CH3 domains and a hinge region that are derived from different antibody isotypes, i.e., a hybrid constant region. For example, in some disclosure, the constant region contains CH2 and/or CH3 domains derived from IgG2 or IgG4 and a mutant hinge region derived from IgG1. Alternatively, a mutant hinge region from another IgG subclass is used in a hybrid constant region. For example, a mutant form of the IgG4 hinge that allows efficient disulfide bonding between the two heavy chains can be used. A mutant hinge can also be derived from an IgG2 hinge in which the first two cysteines are each mutated to another amino acid. Assembly of such hybrid constant regions has been described in U.S. Patent Publication No. 2003/0044423.

In accordance with the present disclosure, the constant region can contain one or more mutations described herein. The combinations of mutations in the Fc portion can have additive or synergistic effects on the prolonged serum half-life and increased in vivo potency of the bifunctional molecule. Thus, in some exemplary disclosure, the constant region can contain (i) a region derived from an IgG sequence in which the Leu-Ser-Leu-Ser (SEQ ID NO: 19) amino acid sequence is replaced with an Ala-Thr-Ala-Thr (SEQ ID NO: 20) amino acid sequence; (ii) a C-terminal alanine residue instead of lysine; (iii) a CH2 domain and a hinge region that are derived from different antibody isotypes, for example, an IgG2 CH2 domain and an altered IgG1 hinge region; and (iv) a mutation that eliminates the glycosylation site within the IgG2-derived CH2 domain, for example, a Gln-Ala-Gln-Ser (SEQ ID NO: 16) amino acid sequence instead of the Gln-Phe-Asn-Ser (SEQ ID NO: 15) amino acid sequence within the IgG2-derived CH2 domain.

### Antibody fragments

The proteins and polypeptides of the disclosure for use in the combination therapy of the disclosure can also include antigen-binding fragments of antibodies. Exemplary antibody fragments include scFv, Fv, Fab, F(ab')₂, and single domain VHH fragments such as those of camelid origin.

Single-chain antibody fragments, also known as single-chain antibodies (scFvs), are recombinant polypeptides which typically bind antigens or receptors; these fragments contain at least one fragment of an antibody variable heavy-chain amino acid sequence (V_{H}) tethered to at least one fragment of an antibody variable light-chain sequence (V_{L}) with or without one or more interconnecting linkers. Such a linker may be a short, flexible peptide selected to assure that the proper three-dimensional folding of the V_{L} and V_{H} domains occurs once they are linked so as to maintain the target molecule binding-specificity of the whole antibody from which the single-chain antibody fragment is derived. Generally, the carboxyl terminus of the V_{L} or V_{H} sequence is covalently linked by such a peptide linker to the amino acid terminus of a complementary V_{L} and V_{H} sequence. Single-chain antibody fragments can be generated by molecular cloning, antibody phage display library or similar techniques. These proteins can be produced either in eukaryotic cells or prokaryotic cells, including bacteria.

Single-chain antibody fragments contain amino acid sequences having at least one of the variable regions or CDRs of the whole antibodies described in this specification, but are lacking some or all of the constant domains of those antibodies. These constant domains are not necessary for antigen binding, but constitute a major portion of the structure of whole antibodies. Single-chain antibody fragments may therefore overcome some of the problems associated with the use of antibodies containing part or all of a constant domain. For example, single-chain antibody fragments tend to be free of undesired interactions between biological molecules and the heavy-chain constant region, or other unwanted biological activity. Additionally, single-chain antibody fragments are considerably smaller than whole antibodies and may therefore have greater capillary permeability than whole antibodies, allowing single-chain antibody fragments to localize and bind to target antigen-binding sites more efficiently. Also, antibody fragments can be produced on a relatively large scale in prokaryotic cells, thus facilitating their production. Furthermore, the relatively small size of single-chain antibody fragments makes them less likely than whole antibodies to provoke an immune response in a recipient.

Fragments of antibodies that have the same or comparable binding characteristics to those of the whole antibody may also be present. Such fragments may contain one or both Fab fragments or the F(ab')₂ fragment. The antibody fragments may contain all six CDRs of the whole antibody, although fragments containing fewer than all of such regions, such as three, four or five CDRs, are also functional.

### Protein production

The antibody-cytokine trap proteins are generally produced recombinantly, using mammalian cells containing a nucleic acid engineered to express the protein. Although one example of a suitable cell line and protein production method is described in Examples 1 and 2, a wide variety of suitable vectors, cell lines and protein production methods have been used to produce antibody-based biopharmaceuticals and could be used in the synthesis of these antibody-cytokine trap proteins.

### Therapeutic indications

This disclosure relates to a combination therapy for the treatment of cancer, or reduction in tumor growth, particularly to a combination of (i) a bifunctional molecule comprising a TGFβRII or fragment thereof capable of binding TGFβ and an antibody, or antigen binding fragment thereof, that binds to an immune checkpoint protein, such as Programmed Death Ligand 1 (PD-L1) and (ii) at least one additional anti-cancer therapeutic agent. The anti-cancer therapeutic agents include, for example, radiation, chemotherapeutic agents, biologics, or vaccines. In certain disclosure herein, the combination therapy provides for a synergistic anti-cancer effect.

Exemplary cancers include colorectal, breast, ovarian, pancreatic, gastric, prostate, renal, cervical, myeloma, lymphoma, leukemia, thyroid, endometrial, uterine, bladder, neuroendocrine, head and neck, liver, nasopharyngeal, testicular, small cell lung cancer, non-small cell lung cancer, melanoma, basal cell skin cancer, squamous cell skin cancer, dermatofibrosarcoma protuberans, Merkel cell carcinoma, glioblastoma, glioma, sarcoma, mesothelioma, and myelodysplastic syndromes.

The cancer or tumor to be treated with an anti-PD-Ll/ TGFβ Trap, in combination with one or more additional anti-cancer therapeutic reagents, such as chemotherapy and/or radiation therapy, may be selected based on the expression or elevated expression of PD-L1 and TGFβ in the tumor, the correlation of their expression levels with prognosis or disease progression, and preclinical and clinical experience on the sensitivity of the tumor to treatments targeting PD-L1 and TGFβ. Such cancers or tumors include but are not limited to colorectal, breast, ovarian, pancreatic, gastric, prostate, renal, cervical, bladder, head and neck, liver, non-small cell lung cancer, melanoma, Merkel cell carcinoma, and mesothelioma.

### Pharmaceutical compositions

The present disclosure also features pharmaceutical compositions that contain a therapeutically effective amount of a protein described herein for use in the therapeutic methods of the disclosure. The composition can be formulated for use in a variety of drug delivery systems. One or more physiologically acceptable excipients or carriers can also be included in the composition for proper formulation. Suitable formulations for use in the present disclosure are found in Remington's Pharmaceutical Sciences, Mack Publishing Company, Philadelphia, Pa., 17th ed., 1985. For a brief review of methods for drug delivery, see, *e.g.*, Langer (Science 249:1527-1533, 1990).

The pharmaceutical compositions are intended for parenteral, intranasal, topical, oral, or local administration, such as by a transdermal means, for therapeutic treatment. The pharmaceutical compositions can be administered parenterally (e.g., by intravenous, intramuscular, or subcutaneous injection), or by oral ingestion, or by topical application or intraarticular injection at areas affected by the vascular or cancer condition. Additional routes of administration include intravascular, intra-arterial, intratumor, intraperitoneal, intraventricular, intraepidural, as well as nasal, ophthalmic, intrascleral, intraorbital, rectal, topical, or aerosol inhalation administration. Thus, the disclosure provides compositions for parenteral administration that comprise the above mention agents dissolved or suspended in an acceptable carrier, preferably an aqueous carrier, e.g., water, buffered water, saline, PBS, and the like. The compositions may contain pharmaceutically acceptable auxiliary substances as required to approximate physiological conditions, such as pH adjusting and buffering agents, tonicity adjusting agents, wetting agents, detergents and the like. The disclosure also provides compositions for oral delivery, which may contain inert ingredients such as binders or fillers for the formulation of a tablet, a capsule, and the like. Furthermore, this disclosure provides compositions for local administration, which may contain inert ingredients such as solvents or emulsifiers for the formulation of a cream, an ointment, and the like.

These compositions may be sterilized by conventional sterilization techniques, or may be sterile filtered. The resulting aqueous solutions may be packaged for use as-is, or lyophilized, the lyophilized preparation being combined with a sterile aqueous carrier prior to administration. The pH of the preparations typically will be between 3 and 11, more preferably between 5 and 9 or between 6 and 8, and most preferably between 7 and 8, such as 7 to 7.5. The resulting compositions in solid form may be packaged in multiple single dose units, each containing a fixed amount of the above-mentioned agent or agents, such as in a sealed package of tablets or capsules. The composition in solid form can also be packaged in a container for a flexible quantity, such as in a squeezable tube designed for a topically applicable cream or ointment.

### Treatments

Determining the dosage and duration of treatment according to any aspect of the present disclosure is well within the skills of a professional in the art. The skilled artisan is readily able to monitor patients to determine whether treatment should be started, continued, discontinued or resumed. The amount of the antibody-TGFβ trap, the anti-cancer therapeutic, or dosage of radiation, for carrying out the combination treatment methods of the disclosure will vary depending on factors such as the condition being treated, the overall health of the patient, and the method, route and dose of administration.

According to certain disclosure herein the antibody-TGFβ trap, and the at least one additional anti-cancer agent, is administered at a therapeutic amount known to be used for treating the specific type of cancer. According to other disclosure herein, due to the observed synergistic effects associated with the combination therapy of the disclosure, the antibody-TGFβ trap, and the at least one additional anti-cancer agent can be administered in an amount that is lower than the therapeutic amount known to be used in monotherapies for treating the cancer.

The optimal dose of the antibody-TGFβ trap is based on the percent receptor occupancy by the antibody moiety to achieve maximal therapeutic effect because the cytokine trap is used in a large excess. For example, the therapeutic dose for a monoclonal antibody targeting a cellular receptor is determined such that the trough level is around 10 to 100 µg/ml, *i.e.*, 60 to 600 nM (for antibody with a dissociation constant (K_{D}) of 6 nM, this trough level would ensure that between 90 to 99% of the target receptors on the cells are occupied by the antibody). This is in large excess of cytokines, which are typically present in pg to ng/ml in circulation.

The optimal dose of antibody-TGFβ trap polypeptide for use in the therapeutic methods of the disclosure will depend on the disease being treated, the severity of the disease, and the existence of side effects. The optimal dose can be determined by routine experimentation. For parenteral administration a dose between 0.1 mg/kg and 100 mg/kg, alternatively between 0.5 mg/kg and 50 mg/kg, alternatively, between 1 mg/kg and 25 mg/kg, alternatively, between 10 mg/kg and 25 mg/kg, alternatively, between 5 mg/kg and 20 mg/kg, alternatively between 2 mg/kg and 10 mg/kg, alternatively, between 5 mg/kg and 10 mg/kg, is administered and may be given, for example, once weekly, once every other week, once every third week, or once monthly per treatment cycle. In some disclosure herein, the effective dose of the antibody-TGFβ trap required to achieve a therapeutic effect in combination therapies will be less than that required in an antibody-TGFβ trap monotherapy to achieve a similar therapeutic effect.

In some disclosure herein, the effective dose will be about 2-10 times less than that required in an antibody-TGFβ trap monotherapy to achieve a similar therapeutic effect. In other disclosure, the effective dose will be about 2-5 times less than that required in an antibody-TGFβ trap monotherapy to achieve a similar therapeutic effect.

The effective dosage of the additional chemotherapeutic reagent, or radiation therapy, for use in combination with an antibody-TGFβ trap for treatment of cancer may vary depending on the particular compound or pharmaceutical composition employed, the mode of administration, the condition being treated and the severity of the condition being treated. A physician or clinician of ordinary skill can readily determine the effective amount of each additional chemotherapeutic reagent, or radiation, necessary to treat or prevent the progression of the cancer. In some disclosure herein, the effective dose of the additional chemotherapeutic reagent or radiation therapy required to achieve a therapeutic effect in the combination therapy of the disclosure will be less than that required in chemotherapeutic or radiation monotherapies to achieve a similar therapeutic effect.

According to the methods of the disclosure, chemotherapeutic agents can be administered in combination with an antibody-cytokine trap molecule to treat cancer or reduce tumor growth. Such chemotherapeutic agents include, for example, alkylating agents, antimetabolites, anthracyclines, plant alkaloids, topoisomerase inhibitors, antineoplastic antibiotics, hormonal agents, anti-angiogenic agents, differentiation inducing agents, cell growth arrest inducing agents, apoptosis inducing agents, cytotoxic agents and other anti-tumor agents. Such drugs may affect cell division or DNA synthesis and function in some way. Representative chemotherapeutic agents include, but are not limited to alkylating agents (such as cisplatin, carboplatin, oxaliplatin, mechlorethamine, cyclophosphamide, chlorambucil, dacarbazine, lomustine, carmustine, procarbazine, chlorambucil and ifosfamide), antimetabolites (such as fluorouracil (5-FU), gemcitabine, methotrexate, cytosine arabinoside, fludarabine, and floxuridine), antimitotics (including taxanes such as paclitaxel and decetaxel and vinca alkaloids such as vincristine, vinblastine, vinorelbine, and vindesine), anthracyclines (including doxorubicin, daunorubicin, valrubicin, idarubicin, and epirubicin, as well as actinomycins such as actinomycin D), cytotoxic antibiotics (including mitomycin, plicamycin, and bleomycin), and topoisomerase inhibitors (including camptothecins such as irinotecan and topotecan and derivatives of epipodophyllotoxins such as amsacrine, etoposide, etoposide phosphate, and teniposide).

In certain disclosure herein, platinum-based therapeutics such as cisplatin, carboplatin and oxaliplatin are utilized. Other anti-cancer agents whose treatment and effects can benefit from combination with anti-PD-L1/TGFβ Trap molecule include antimetabolites, such as flurouracil (5-FU), which interfere with DNA synthesis. In certain disclosure herein, combinations of one or more chemotherapeutic agents may be administered with the anti-PD-L1/TGFβ Trap molecule. In other disclosure herein, combinations of one or more chemotherapeutic agents may be administered with and radiation therapy and the anti-PD-L1/TGFβ Trap molecule.

In a specific disclosure herein, oxaliplatin may be administered in a dose of between 20 mg/m² and 200 mg/m², alternatively between 40 mg/m² and 160 mg/m², alternatively, between 60 mg/m² and 145 mg/m², alternatively, between 85 mg/m² and 135 mg/m², alternatively between 40 mg/m² and 65 mg/m².

In a specific disclosure herein, 5-FU may be administered in a dose of between 100 mg/m² and 3000 mg/m², alternatively, between 250 mg/m² and 2400 mg/m², alternatively, between 400 mg/m² and 1500 mg/m², alternatively, between 200 mg/m² and 600 mg/m². Indisclosure herein, the 5-FU dose may be administered, for example, by infusion over an extended period of time.

In a specific disclosure herein, leucovorin may also be administered, to enhance the effects of the 5-FU or to decrease the side effects associated with chemotherapy.

In a disclosure herein, the following chemotherapeutic regimen is provide as an example for use in combination with the anti-PD-L1/TGFβ Trap molecule. On day 1, 85 mg/m² of oxaliplatin and 200 mg/m² of leucovorin are administered followed 2 hours later by administration of 400 mg/m² bolus of 5-FU and 600 mg/m² infusion of 5-FU. On day 2, 200 mg/m² of leucovorin is administered followed 2 hours later by 400 mg/m² bolus of 5-FU and 600 mg/m² infusion of 5-FU.

In another disclosure herein, the chemotherapeutic regimen includes, for example, administration on day 1 of a 85 mg/m² dose of oxaliplatin, a 400 mg/m² dose of leucovorin, a 400 mg/m² IV bolus dose of 5-FU and a 600 mg/m² infusion of 5-FU followed by 1200 mg/m²/day × 2 days (total 2400 mg/ml² over 46-48 hours) IV continuous infusion. The treatment is repeated every 2 weeks.

In another disclosure herein, a 2 hour infusion of 400 mg/m² of leucovorin is administered followed by a 5-FU 46-hour infusion of 2400mg/m². Oxaliplatin is also infused for two hours on day 1 at a dose of 130 mg/m². The treatment is repeated every two weeks.

According to the methods of the present disclosure, radiation can be administered in combination with an antibody-cytokine trap molecule to treat cancer. Radiation therapy typically uses a beam of high-energy particles or waves, such as X-rays and gamma rays, to eradicate cancer cells by inducing mutations in cellular DNA. Cancer cells divide more rapidly than normal cells, making tumor tissue more susceptible to radiation than normal tissue. Any type of radiation can be administered to a patient, so long as the dose of radiation is tolerated by the patient without significant negative side effects. Suitable types of radiotherapy include, for example, ionizing radiation (e.g., X-rays, gamma rays, or high linear energy radiation). Ionizing radiation is defined as radiation comprising particles or photons that have sufficient energy to produce ionization, *i.e.*, gain or loss of electrons. The effects of radiation can be at least partially controlled by the clinician. The dose of radiation is preferably fractionated for maximal target cell exposure and reduced toxicity. Radiation can be administered concurrently with radiosensitizers that enhance the killing of tumor cells, or with radioprotectors (e.g., IL-1 or IL-6) that protect healthy tissue from the harmful effects of radiation. Similarly, the application of heat, *i.e.*, hyperthermia, or chemotherapy can sensitize tissue to radiation.

The source of radiation can be external or internal to the patient. External radiation therapy is most common and typically involves directing a beam of high-energy radiation (a particle beam) to a tumor site through the skin using, for instance, a linear accelerator. While the beam of radiation is localized to the tumor site, it is nearly impossible to avoid exposure of normal, healthy tissue. However, external radiation is usually well tolerated by patients.

In another example, radiation is supplied externally to a patient using gamma rays. Gamma rays are produced by the breakdown of radioisotopes such as cobalt 60. Using a treatment approach called Stereotactic Body Radiation Therapy (SBRT), gamma rays can be tightly focused to target tumor tissue only, such that very little healthy tissue is damaged. SBRT can be used for patients with localized tumors. On the other hand, X-rays, produced by a particle accelerator, can be used to administer radiation over a larger area of the body.

Internal radiation therapy involves implanting a radiation-emitting source, such as beads, wires, pellets, capsules, etc., inside the body at or near the tumor site. The radiation used comes from radioisotopes such as, but not limited to, iodine, strontium, phosphorus, palladium, cesium, iridium, phosphate or cobalt. Such implants can be removed following treatment, or left in the body inactive. Types of internal radiation therapy include, but are not limited to, brachytherapy, interstitial irradiation, and intracavity irradiation. A currently less common form of internal radiation therapy involves biological carriers of radioisotopes, such as with radioimmunotherapy wherein tumor-specific antibodies bound to radioactive material are administered to a patient. The antibodies bind tumor antigens, thereby effectively administering a dose of radiation to the relevant tissue.

Radiation therapy is useful as a component of a regimen to control the growth of a primary tumor (see, *e.g.* Comphausen et al. (2001) "Radiation Therapy to a Primary Tumor Accelerates Metastatic Growth in Mice," Cancer Res. 61:2207-2211). Although radiation therapy alone may be less effective at treating cancer, combining radiation with an anti-PD-L1/TGFβ Trap molecule as described herein, can enhance the local and systemic efficacy of radiation therapy.

Because radiation kills immune effector cells, the dose and timing of the radiation is important. T cells and dendritic cells in an irradiated tumor decrease immediately after irradiation; however, T-cell levels rebound higher than baseline levels. No matter the method of administration, a complete daily dose of radiation can be administered over the course of one day. Preferably, the total dose is fractionated and administered over several days. Accordingly, a daily dose of radiation will comprise approximately 1-50 Gy/day, for example, at least 1, at least 2, at least 3, 1-4, 1-10, 1-20, 1-50, 2-4, 2-10, 2-20, 2-25, 2-50, 3-4, 3-10, 3-20, 3-25, 3-50 Gy/day.

The daily dose can be administered as a single dose, or can be a "microfractionated" dose administered in two or more portions over the course of a day. When internal sources of radiation are employed, e.g., brachytherapy or radio-immunotherapy, the exposure time typically will increase, with a corresponding decrease in the intensity of radiation.

According to some disclosure herein of the disclosure, the antibody-TGFβ trap and the at least one additional anti-cancer agent are administered simultaneously. According to another disclosure herein, the antibody-TGFβ trap and the at least one additional anti-cancer agent are administered sequentially.

The dosing frequency of the antibody-TGFβ trap and the at least one additional anti-cancer therapeutic agent may be adjusted over the course of the treatment, based on the judgment of the administering physician.

### EXAMPLES

The invention now being generally described, will be more readily understood by reference to the following examples, which are included merely for purposes of illustration of certain aspects and embodiments of the present invention, and are not intended to limit the scope of the invention in any way.

### EXAMPLE 1 ― DNA construction and protein expression

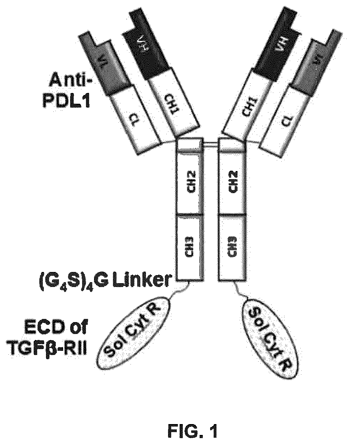

Anti-PD-L1/TGFβ Trap is an anti-PD-L1 antibody-TGFβ Receptor II fusion protein. The light chain of the molecule is identical to the light chain of the anti-PD-L1 antibody (SEQ ID NO:1). The heavy chain of the molecule (SEQ ID NO:3) is a fusion protein comprising the heavy chain of the anti-PD-L1 antibody (SEQ ID NO:2) genetically fused to via a flexible (Gly₄Ser)₄Gly linker (SEQ ID NO:11) to the N-terminus of the soluble TGFβ Receptor II (SEQ ID NO:10). At the fusion junction, the C-terminal lysine residue of the antibody heavy chain was mutated to alanine to reduce proteolytic cleavage. For expression of anti-PD-L1/TGFβ Trap, the DNA encoding the anti-PD-L1 light chain (SEQ ID NO:4) and the DNA encoding the anti-PD-L1/TGFβ Receptor II (SEQ ID NO:5) in either the same expression vector or separate expression vectors were used to transfect mammalian cells using standard protocols for transient or stable transfection. Conditioned culture media were harvested and the anti-PD-L1/TGFβ Trap fusion protein was purified by standard Protein A Sepharose chromatography. The purified protein comprising one anti-PD-L1 antibody and two soluble TGFβ Receptor II molecules (FIG. 1) has an estimated molecular weight (MW) of about 190 kilodaltons on size exclusion chromatography and SDS-polyacrylamide electrophoresis under non-reducing conditions. Under reducing conditions, the light and heavy chains have apparent MW of 28 and 75 kilodaltons, respectively.

The anti-PD-L1(mut)/TGFβ Trap fusion protein, which contains an analogous heavy chain fusion polypeptide (SEQ ID NO:7) and a light chain with the mutations A31G, D52E, R99Y in the variable region that abrogate the binding to PD-L1 (SEQ ID NO:6), was similarly prepared. It was used in subsequent experiments as a TGFβ Trap control.

### EXAMPLE 2 ― Production of anti-PD-L1/TGFβ Trap as a biotherapeutic

The anti-PD-L1/TGFβ Trap produced by transient transfection of human embryonic kidney 293 (HEK) cells was found to contain varying degrees of a clipped species, which appeared as a faint band with an apparent MW of about 60 kD on SDS-PAGE under reducing conditions. This band was confirmed to be the heavy chain of the anti-PD-L1/TGFβ Trap cleaved at a site in the N-terminal portion of TGFβRII close to the fusion junction.

Stable clones expressing anti-PD-L1/TGFβ Trap were generated in the CHO-S host cell line, which was pre-adapted for growth in serum-free media in suspension culture. Cells were transfected with an expression vector containing a gene encoding the anti-PD-Ll-TGFβRII protein and a glutamine synthetase selection marker. Subsequent selection of stable integrants was made with L-methionine sulfoximine (MSX). Anti-PD-L1/TGFβ Trap expressing cell lines were generated using a minipool approach, followed by the deposition of single cells into 384-well plates, using a Beckton-Dickinson fluorescence activated cell sorter (FACS Aria II). Growth, productivity, and protein quality were evaluated in a generic platform fed-batch assay. Based on these analyses, 14 clones were selected as lead candidates for further studies. A stability study with the clones was carried out to ~90 PDL (population doubling level) from research cell banks established during scale up of clones. At the conclusion of mini-pool development it was discovered that the heavy chain-linker-TGFβRII subunit underwent clipping, as was seen in transient expression. All clones in the stability study produced the clipped species, although it was shown in the protein A-purified material that the percent clipped species relative to the intact subunit varied with each clone. In addition, an improved purification process consisting a protein A chromatography followed by strong cation exchange was developed to reduce co-purification of the clipped species. Even with the improved process, purified material with the required final levels of clipped species of <5% could only be achieved using clones producing low levels of clipping. Based on these combined analyses, clone 02B15 was selected as the final candidate clone. Analysis of anti-PD-L1/TGFβ Trap expressed by this clone at zero PDL, thirty PDL, sixty PDL and ninety PDL shows that the percentage of clipping did not increase with population doubling levels.

### REFERENCE EXAMPLE 3 ― Combination of Chemotherapy and anti-PD-L1/TGFβ Trap in a subcutaneous MC38 tumor mouse model

Colorectal cancer (CRC) is the third most common cancer in males and the second in females, with over 1.2 million new cases worldwide. Despite significant progress in treatment over the last decade, CRC is the fourth most common cause of cancer-related deaths. Thus, novel treatment modalities are needed. In the working example set forth below, the efficacy of the anti-PD-L1/TGFβ Trap molecule was investigated in combination with oxaliplatin (Ox) and 5-fluorouracil (5-FU) based therapy in a murine model of colorectal cancer.

Combination treatment with anti-PD-L1/TGFβ Trap and the chemotherapeutic reagent Ox/5-FU in mice with subcutaneous MC38 tumors resulted in significant inhibition of tumor growth. These preclinical data support a strategy of combining chemotherapy (Ox/5-FU) with anti-PD-L1/TGFβ Trap immunotherapy for the treatment of colorectal cancer in the clinic.

### Materials and Methods

The MC38 tumor cell line was obtained from American Type Culture Collection (ATCC). TheMC38 cell line was tested and verified to be free of adventitious viruses and mycoplasma. C57BL/6 mice, 8-12 weeks of age, were obtained from Charles River Laboratories. B6.129S2-Ighm^{tmlCgn}/J mice, 8-12 weeks of age, were from Jackson Laboratories.

Test material doses were as follows: Anti-PD-L1/TGFβ Trap: 24.6 mg/kg; 492 µg/mouse; 2.46 mg/mL; 0.2 mL dose volume administered intravenously. Fluorouracil (5-FU): 60.0 mg/kg; 120 µg/mouse; 6.00 mg/mL; 0.02 mL dose volume administered intravenously. Oxaliplatin: 5.0 mg/kg; 10 µg/mouse 0.500 mg/mL 0.02 mL administered i.p. The value in mg/kg was approximate, assuming an average body weight of 20 g per mouse.

The negative control was an inactive isotype control (Anti-PD-L1(mut)) administered at a test concentration of 400 µg/mouse.

Cell Culture. MC38 cells were cultured under aseptic conditions in Dulbecco's minimal essential medium (DMEM) containing 10% heat-inactivated fetal bovine serum and maintained at 37°C and 5% CO². Cells were passaged upon reaching 50-70% confluence at a ratio of 1:5, for a total of 2 passages prior to in vivo implantation. Cells were harvested by trypsinization and viable cell counts were determined using a hemocytometer and trypan blue exclusion staining. All cell culture reagents were purchased from Life Technologies (Gaithersburg, MD).

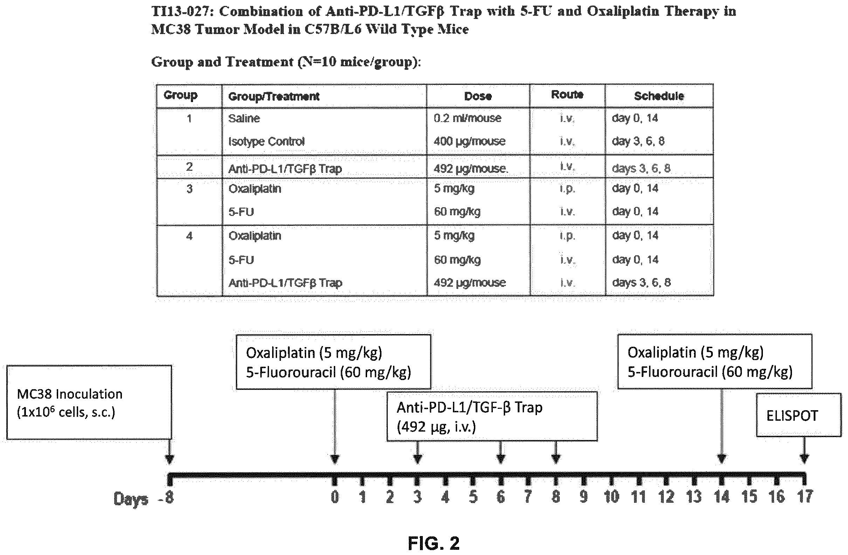

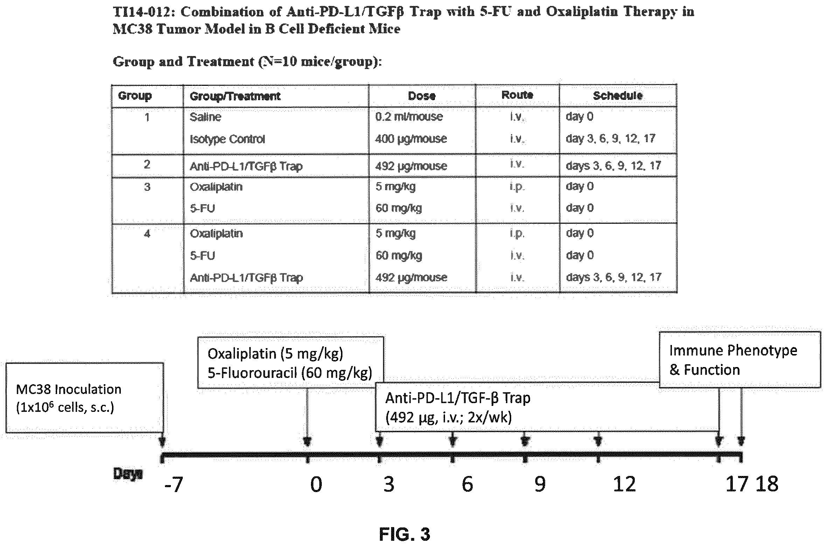

MC38 Tumor Model. In study TI13-027, MC38 tumor cells (1×10⁶ cells/mouse) were suspended in 100 µL of sterile PBS and implanted into right flank of C57BL/6 mice. When the tumor sizes reached an average of~45 mm³, the mice were randomized into 4 groups (N=10 mice/group) to initiate therapy. Treatments were administered as per the dose schedules as set forth in FIG. 2 and FIG. 3. The length (L), width (W) and height (H) of tumor was measured with digital caliper and recorded automatically to computer twice per week using WinWedge software. Body weights were also recorded twice per week to assess tolerability. Tumor volumes were calculated by the Ellipsoid volume formulas: Volume = π/6^{∗}(L × W × H); where L = length, W = width and H = height of the tumor. Efficacy was determined by measuring tumor volume throughout the duration of the *in vivo* study and the tumor weights were measured at study termination point as described below. All animals were sacrificed on day 17 and tumors were excised and weighed. The spleens were harvested for IFN-γ ELISPOT analyses.

In study TI14-012, MC38 tumor cells were injected into B6.129S2-Ighm^{tm1Cgn}/J mice as described above. All other procedures for evaluation of tumor growth and treatment efficacy were also as described above.

IFN-γ ELISpot Assay. The enzyme-linked immunosorbent spot (ELISpot) assay was used to measure the cytotoxic T lymphocytes (CTL) response against the p15E antigen, which is a known T cell rejection epitope in MC38 tumors (Yang and Perry-Lalley J Immunotherapy 2000; 23:177-183). The ELISpot assay measures the frequency of IFN-γ producing CD8⁺ T cells following co-culture with antigen presenting cells (APC) loaded with the p15E epitope KPSWFTTL. APCs loaded with an irrelevant peptide, SIINFEKL, derived from chicken ovalbumin (OVA) served as a negative control. Positive control samples were stimulated with PMA and ionomycin, which triggers a non-specific activation of cytotoxic T lymphocytes. ELISpot assay was performed using a mouse IFN-γ ELISpot Kit from BD Biosciences according to the manufacturer's instructions. On day 17 of study TI13-027, the spleens of N=5 mice/group were harvested, processed into single cell suspensions, stimulated with P15E peptide at a final concentration of 1 µg/mL, and then cultured at 37°C for 7 days. After the in vitro stimulation, CD8⁺ T cells were isolated by magnet activated cell sorting using the CD8⁺ T cell isolation kit (Miltenyi Biotech) and the AutoMACS Pro Separator. To establish the co-culture system for the *in vitro* stimulation ELISpot assay, APCs derived from naive mouse splenocytes were pulsed with the KPSWFTTL peptide or the irrelevant SIINFEKL peptide for one hour and then irradiated with 2 Gy in the GammaCell 40 Exactor. Isolated CD8⁺ T cells (1×10⁵ cells/well) from experimental mice were cultured in triplicate in ELISpot assay plates (anti-IFN-y antibody coated) with peptide-pulsed and irradiated APCs (2.5×10⁵ cells/well).

On day 18 of study TI14-012, an *ex vivo* ELISpot assay was established in which the spleens of N=5 mice/group were harvested, processed into single cell suspensions, and CD8⁺ T cells were isolated with magnet activated cell sorting using the CD8⁺ T cell isolation kit (Miltenyi Biotech) and the AutoMACS Pro Separator. To establish the co-culture system for the *ex vivo* ELISpot assay, APCs derived from naive mouse splenocytes were pulsed with the KPSWFTTL peptide or the irrelevant SIINFEKL peptide for one hour and then irradiated with 2 Gy in the GammaCell 40 Exactor. Isolated CD8⁺ T cells (5×10⁵ cells/well) from experimental mice were cultured in triplicate in ELISpot assay plates (anti-IFN-y antibody coated) with peptide-pulsed and irradiated APCs (5×10⁵ cells/well).

In both experiments, the experimental CD8+ T cells were co-cultured with peptide-pulsed APCs at 37°C for 19-20 hours prior to being removed from the assay plate. A biotinlyated anti-IFN-y antibody was added to each well of the plate, followed by a wash step, and then addition of a streptavidin-HRP detection conjugate. After another wash step, the plate was incubated with a chromogenic substrate solution; the reaction was monitored and then stopped by rinsing the plate with water. The number of IFN-γ positive spots in each well of the assay plate was counted using a CTL-Immunospot S5UV Analyzer (Cellular Technology Limited). The data are represented as the mean number of spots/well ± SEM.

Mortality checks were performed once daily during the study. Clinical Observations. Clinical signs (such as ill health and behavioral changes) were recorded for all animals once daily during the study using the body condition (BC) scoring system as previously described (Ullman-Cullere and Foltz, Lab Anim Sci. 1999; 49:319-23). Moribund mice were humanely euthanized by CO² asphyxiation. Body weights for all animals on the study were recorded twice per week including the termination day of each group. Tumors volumes were measured in three dimensions with digital calipers and recorded automatically to a computer twice per week using WinWedge software for the duration of the experiment. Tumor volumes were calculated using the ellipsoid volume formula: Volume =0.5236 (L × W × H); where L = length, W = width and H = height of the tumor. At the time of sacrifice, the primary tumor was excised and weighed as a secondary efficacy endpoint. The frequency of IFN-γ producing, P15E-specific CD8⁺ T cells was quantified by ELISpot assay. Mouse IFN-γ ELISpot assays were performed using a mouse IFN-γ ELISpot kit (BD Biosciences) according to the manufacturer's instructions.

Statistical Analysis. Tumor volumes were measured twice per week throughout the study period. Tumor volume data was presented as the mean ± standard error of the mean (SEM). The tumor growth inhibition % T/C ratio was calculated as the tumor volume of the treatment group divided by the tumor volume of control group and then multiplied 100. Tumor volume data was log transformed and two-way, repeated measures ANOVA with Tukey's correction for multiple comparisons was performed to measure statistical differences between treatment groups. T/C was calculated as the tumor volume of the treatment group divided by the tumor volume of control group. Tumor weights were measured at study completion. The data was represented as the mean ± SEM. The % T/C ratio was calculated as the tumor weight of the treatment group divided by the tumor weight of control group and then multiplied 100. Tumor weight data was evaluated with one-way ANOVA with Tukey's correction for multiple comparisons to measure statistical differences between treatment groups. IFN-γ ELISpot data was expressed as the mean ± SEM. A One-Way ANOVA with Tukey's correction for multiple comparisons was used for statistical analyses using GraphPad Prism Software. p<0.05 was determined to be statistically significant.

### Results

Due to the immunogenicity caused by the fully humanized antibody in B cell competent mice, the anti-PD-L1/TGFβ Trap molecule could only be administered three times within one week in the C57BL/6 wild-type mice in study TI13-027. Consequently, significant antitumor activity was not observed with anti-PD-L1/TGFβ Trap monotherapy (% T/C = 91% in tumor volume; see Figure 4). The Oxaliplatin/5-FU treatment induced significant tumor growth inhibition in theMC38 subcutaneous tumor model compared to the Isotype control (% T/C = 53.2% in tumor volume; p<0.0001). Combination therapy with anti-PD-L1/TGFβ Trap and oxaliplatin/5-FU significantly inhibited MC38 tumor growth compared with the control group (% T/C = 33.2% in tumor volume; p<0.0001). Moreover, the combination of anti-PD-L1/TGFβ Trap and oxaliplatin/5-FU significantly improved tumor growth control relative to oxaliplatin/5-FU alone (439.6 mm³ vs. 703.7 mm³ in tumor volume; p<0.0001). The same trend was observed in which the combination treatment produced statistically significant improvements in tumor growth control compared to anti-PD-L1/TGFβ Trap alone (439.6 mm³ vs. 1204.0 mm³; p<0.0001) (see Fig. 4A-D and Table 1).

**Table 1 Results of Tumor Volume, Tumor Weight, and ELISpot Assay at Study Completion (C57BL/6 mice; Study TI13-027)**

| **Group** | **Treatment** | **Tumor volume (mm³)*** | **% T/C of tumor volume** | **Tumor weight (mg)*** | **% TIC of tumor weight** | **IFN-γ ELISPOT** |
|---|---|---|---|---|---|---|
| G1 | Isotype Control (Anti-PD-L1(mut) | 1323.5 ± 199.1 | 100 | 1448.5 ± 220.0 | 100 | 49.0 ± 4.0 |
| G2 | Anti-PD-L1/TGFβ Trap | 1204.0 ± 217.2 | 91 | 1196.8 ± 248.8 | 82.6 | 113.3 ± 4.5 |
| G3 | Oxaliplatin + 5-FU | 703.7 ± 115.6 | 53.2 | 701.4 ± 102.8 | 48.4 | 108.3 ± 23.6 |
| G4 | Oxaliplatin/5-FU + Anti-PD-L1/TGFβ Trap | 439.6 ± 71.1 | 33.2 | 438.2 ± 71.9 | 30.3 | 258.0 ± 14.3 |

Finally, anti-PD-L1/TGFβ Trap monotherapy or the oxaliplatin/5-FUmonotherapy were observed to significantly increase the frequency of IFN-γ producing CD8⁺ T cells compared to the Isotype control group as measured by ELISpot assay (p<0.05 and p<0.05, respectively). The combination of anti-PD-L1/TGFβ Trap and oxaliplatin/5-FU significantly enhanced the frequency of P15E-specific, IFN-γ producing CD8₊ T cells relative to either monotherapy group (p<0.05; see FIG. 4C).

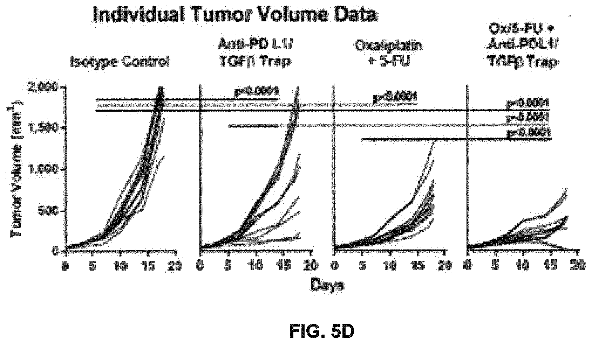

In study TI14-012, utilizing B cell deficient mice to avoid the mouse antibody against human antibody (MAHA) response, experimental animals were treated five times with anti-PDL1/TGFβ Trap. Not surprisingly, greater antitumor activity was observed with anti-PDL1/TGFβ Trap monotherapy (% T/C = 57.3% in tumor volume) compared to study TI13-027 in which wild-type mice were treated only three times (% T/C = 91% in tumor volume). In study TI14-012, the combined treatment with anti-PD-L1/TGFβ Trap and oxaliplatin/5-FU was significantly more effective compared to either of the monotherapies (p<0.0001) or the isotype control (p<0.0001) (see Fig. 5D and Table 2).

**Table 2 Results of Tumor Volume, Tumor Weight, and ELISpot Assay at Study Completion (B6.129S2-Ighm^{tmlCgn}/J mice; Study TI14-012)**

| **Group** | **Treatment** | **Tumor volume (mm³)*** | **% T/C of tumor volume** | **Tumor weight (mg)*** | **% T/C of tumor weight** | **IFN-γ ELISPOT** |
|---|---|---|---|---|---|---|
| G1 | Isotype Control (Anti-PD-L1(mut) | 2003.4 ±122.4 | 100 | 2336.2 ± 1 64.8 | 100 | 51.7 ± 5.5 |
| G2 | Anti-PD-L1/TGFβ Trap | 1147.7 ± 234.9 | 57.3 | 1265.0 ± 256.5 | 54.1 | 160.3 ± 18.5 |
| G3 | Oxaliplatin + 5-FU | 743.9 ± 92.4 | 37.1 | 822.7 ± 86.0 | 35.2 | 107.7 ± 13.0 |
| G4 | Oxaliplatin/5-FU + Anti-PD-L1/TGFβ Trap | 380.8 ± 74.6 | 19.0 | 362.8 ± 70.9 | 15.5 | 369.7 ± 39.7 |

Similarly, anti-PD-L1/TGFβ Trap monotherapy resulted in significantly increased frequencies of IFN-γ producing CD8⁺T cells compared to the isotype control (see Fig. 5C; p<0.05). The combined treatment of anti-PD-L1/TGFβ Trap and oxaliplatin/5-FU resulted in a synergistic increase in the frequency of P15-specific, IFN-γ producing CD8⁺T cells compared to either monotherapy group or the Isotype control (see Fig. 5C; p<0.05).

Anti-PD-L1/TGFβ Trap is a bifunctional antibody-cytokine receptor fusion protein designed to reverse both the cell-intrinsic and extrinsic immune suppression in the tumor microenvironment through dual targeting of the PD-1/PD-L1 axis and TGFβ signaling. In the studies described herein, significant MC38 tumor growth inhibition and the synergistic induction of P15E-specific CD8⁺ T cell IFN-γ production were observed with the combination of anti-PDL1/TGFβ Trap and Ox/5-FU treatment in mice with subcutaneous MC38 tumors. These effects on antitumor efficacy and immune response observed in wild-type mice were accentuated in B cell deficient mice. The difference is believed to be primarily due to administration of a greater number of doses of anti-PD-L1/TGFβ Trap and absence of the MAHA (mouse against human antibody) response in the B cell deficient mice. Taken together, these data demonstrate that components of chemotherapy (Ox/5-FU) can be effectively combined with anti-PDL1/TGFβ Trap therapy to enhance tumor growth inhibition and tumor-reactive CD8⁺ T cell responses in a mouse colorectal cancer model. In conclusion, the preclinical results support a combination for the treatment of colorectal cancer in the clinic.

### EXAMPLE 4 ― Combination of radiation therapy and anti-PD-L1/TGFβ Trap in a Intramuscular MC38 tumor mouse model

The anti-PD-L1/TGFβ Trap molecule is comprised of the extracellular domain of the human TGFβRII (TGFβ Trap) covalently linked to the C-terminus of the heavy chain of a human anti-PD-L1 antibody. Anti-PD-L1/TGFβ Trap monotherapy has shown superior antitumor efficacy in multiple preclinical models. In the studies reported here, we investigated the antitumor activity of the anti-PD-L1/TGFβ Trap in combination with fractionated local radiation therapy in B6.129S2-Ighm^{tm1Cgn}/J mice bearing intramuscular MC38 colorectal tumors. The data showed that the combination of radiation given as four fractionated doses of local radiation (360 rads/dose) and a single administration of anti-PD-L1/TGFβ Trap (55 µg) had remarkably synergistic antitumor effects resulting in tumor remission in 100% of the mice. In addition, the combination of radiation given as four fractionated doses of local radiation (500 rads/dose) and a single administration of the anti-PD-L1/TGFβ Trap (164 µg) elicited anti-cancer effects on tumors at sites distal to the tumor being irradiated, a demonstration of the abscopal effect, and an indication that such treatment would be useful in treating metastasis. By comparison, monotherapy with either radiation or anti-PD-L1/TGFβ Trap treatment alone resulted in a modest reduction in tumor burden. Furthermore, significant increases in the frequency of P15E-specific, IFN- γ producing CD8⁺ T cells were observed in the mice receiving the combination therapy. Finally, the combination therapy was associated with improved infiltration of MC38 tumors by effector CD8⁺ T cells and NK cells. These results indicate that anti-PD-L1/TGFβ Trap treatment synergizes with radiation to facilitate T cell mediated antitumor responses. The results described below support this combination strategy for potential clinical applications.

### Materials and Methods

Cell line: MC38 murine colon carcinoma cell line was a gift from the Scripps Research Institute. The cell line was tested and verified to be murine virus and mycoplasma free. Animals B6.129S2-Ighm^{tm1Cgn}/J mice (C57BL/6), 8-12 weeks of age, were obtained from Jackson Laboratories.

Test material doses were as follows: Anti-PD-L1/TGFβ Trap: 2.75 mg/kg; 55 µg/mouse; 13.75 mg/mL; 0.2 mL dose volume administered intravenously and anti-PD-L1/TGFβ Trap: 8.25 mg/kg; 164 µg/mouse; 41.25 mg/mL; 0.2 mL dose volume administered intravenously.

Negative controls was as follows: inactive isotype control (anti-PD-L1(mut) A11-121-6) was administered at a test concentration of either 133 µg/mouse or 45 µg/mouse.

MC38 cells were cultured under aseptic conditions in Dulbecco's minimal essential medium, containing 10% heat-inactivated fetal bovine serum, and maintained at 37°C and 5% CO2. Cells were passaged upon reaching 50-70% confluence at a ratio of 1:5, for a total of 2 passages prior to in vivo implantation. Cells were harvested by trypsinization and viable cell counts were determined using a hemocytometer and trypan blue exclusion staining.

C38 tumor model: C57BL/6.129S2-Ighmtm1Cgn/J mice were implanted intramuscularly into the right thigh with 0.5×10⁶ viable MC38 tumor cells in 0.1ml PBS on day -8. When the tumors had reached a mean volume of ~128 mm³, mice were randomized into treatment groups. Treatment started on day 0 (8 days after tumor cell inoculation).

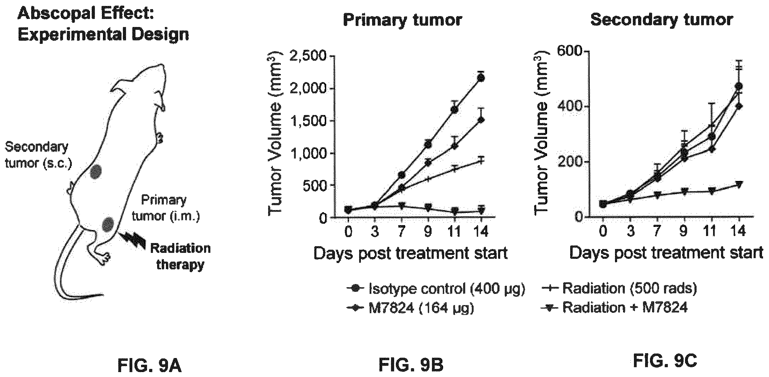

Localized radiation therapy can elicit anti-cancer effects at distal sites, a phenomenon known as an "abscopal" effect. To test the effect of the Anti-PD-L1/TGFβ Trap on the abscopal effect of radiation therapy, 7 days prior to treatment, mice were inoculated with 0.5 × 10⁶ viable MC38 tumor cells to generate a primary, intramuscular MC38 tumor in the right thigh, and with 1 × 10⁶ MC38 cells subcutaneously in the left flank to generate a secondary, subcutaneous MC38 tumor (FIG 9A). Treatment commenced on day 7.

Radiotherapy: Mice were positioned on a dedicated plexiglass tray, and the whole body was protected by lead shielding except for the area of the tumor to be irradiated. Radiotherapy was delivered to the tumor field through the use of GammaCell 40 Exactor.

Enzyme-linked Immunosorbent Spot (ELISpot) Assay: The ELISpot assay was used to measure the cytotoxic T lymphocyte (CTL) response against the p15E antigen, which is a known T cell rejection epitope expressed by MC38 tumors (refer to Yang and Perry-Lalley 2000). The ELISpot assay measures the frequency of IFN-γ producing CD8⁺ T cells following co-culture with antigen presenting cells (APCs) loaded with the p15E epitope KPSWFTTL. A PCs loaded with an irrelevant peptide derived from chicken ovalbumin (SIINFEKL) served as a negative control. Positive control samples were stimulated with PMA and ionomycin, which triggers a non-specific activation of CTLs. The ELISpot assay was performed using a kit from BD Biosciences. On study day 14, the spleen was harvested from one mouse in each study group and processed into a single cell suspension. The CD8⁺ T cells were isolated by magnet activated cell sorting using the CD8⁺ T cell isolation kit from Miltenyi Biotech, and the AutoMACS Pro Separator. CD8⁺ T cells were then seeded in ELISpot assay plates (anti-IFN-y antibody coated) in co-culture with APC derived from naive mouse splenocytes pulsed with the KPSWFTTL peptide for one hour, and then irradiated with 2 Gy in the GammaCell 40 Exactor. After incubation at 37°C for 16-20 hours, the cells were removed from the assay plate. A biotinlyated anti-IFN-y antibody was added to each well of the plate, followed by a wash step, and then addition of a streptavidin-HRP detection conjugate. After another wash step, the plate was incubated with a chromogenic substrate solution; the reaction was monitored and then stopped by rinsing the plate with water. The number of IFN-γ positive spots in each well of the assay plate was measured using an Immunospot ELISpot reader system.

Immune Phenotype: Cell suspensions were prepared from spleens by mechanical disruption followed by lysis of red blood cells. Tumor cell suspensions were prepared by enzymatic digestion of finely minced tumor slurries. Slurries were incubated in a solution of type IV collagenase (400 units/ml) and DNase 1 (100 µg/ml) for one hour at 37°C with frequent agitation. Following tumor digestion, debris was separated by sedimentation, and suspensions were passed through a 40 µm nylon cell strainer. Antibody staining of spleen and tumor cell suspensions for FACS analysis was performed following the manufacturer's recommendations (e.g. eBioscience or BD Biosciences).

For the analysis of spleen samples, a parental gate was created around the lymphocyte population as identified by forward and side scatter characteristics. From the lymphocyte gate, subpopulations of immune cells were identified on dot plots: helper T cells (CD4⁺), cytotoxic T lymphocytes (CD8⁺), NK cells (NK1.1⁺), effector memory CD8⁺ T cells (CD8⁺/CD44high/CD62Llow), central memory CD8⁺ T cells (CD8⁺/CD44high/CD62Lhigh) and regulatory T cells (CD4⁺/CD25⁺/Foxp3⁺). To assess degranulation as a measure of lytic activity, CD107a on the lymphocyte cell surface was measured. Following the staining of cell surface proteins, samples were fixed and permeabilized to allow for intracellular staining of the T-box transcription factors (Eomes and T-bet) and effector cytokines (IFN-γ and Granzyme B). From the leukocyte gate, subpopulations of myeloid cells were identified on dot plots: Dendritic cell (CD11c⁺/I-Ab⁺), neutrophils (CD11b⁺/Ly6G⁺), macrophages +(CD11b⁺/Ly-6Chigh) and MDSCs (Gr-1+/CD11b⁺).

A similar gating strategy was employed for the analysis of tumor samples, with the exception that a parental gate was first created around the CD45⁺ cell population to identify the tumor infiltrating leukocytes from the other tumor cells and stromal components.

Study Design. TI13-109 Combination Therapy of Radiation with Anti-PD-L1/TGFβ Trap in MC38 Model in B-cell Deficient Mice. Group and treatment (N=10).
Part 1: Efficacy

| | | |
|---|---|---|
| 1. Isotype Control | 133 µg i.v. | day 2 |
| 2. Radiation | 360 rads/day | day 0-3 |
| 3. Anti-PD-L1/TGFβ Trap | 55 µg i.v. | day 2 |
| 4. Anti-PD-L1/TGF β Trap | 164 µg i.v. | day 2 |
| 5. Radiation | 360 rads/day | day 0-3 |
| Anti-PD-L1/TGF β Trap | 55 µg i.v. | day 2 |
| 6. Radiation | 360 rads/day | day 0-3 |
| Anti- PD-L1/TGF β Trap | 164 µg i.v. | day 2 |

Part 2: ELISpot Assay: On day 14, all mice were sacrificed and a subset of N=5 mice/group were analyzed for functional responses via ELISpot assay. The spleens were harvested and processed for the ELISpot assay as described above. The number of IFN-γ positive spots in each well of the assay plate was measured using an Immunospot ELISpot reader system.

Study Design: TI14-013 Combination Therapy of Radiation with Anti-PD-L1/TGFβ Trap in MC38 Model in B-cell Deficient Mice. Group and treatment (N=10).
Part 1: Efficacy

| | | |
|---|---|---|
| 1. Isotype Control | 45 µg i.v. | day 2 |
| 2. Radiation | 360 rads/day | day 0-3 |
| 3. Anti-PD-L1/TGFβ Trap | 55 µg i.v. | day 2 |
| 4. Radiation | 360 rads/day | day 0-3 |
| Anti- PD-L1/TGFβ Trap | 55 µg i.v. | day 2 |

Part 2: ELISpot Assay and Immune Phenotype. On day 14, all mice were sacrificed and a subset of N=5 mice/group were analyzed for splenic functional responses via ELISpot assay and immune phenotype of TILs. The spleens were harvested and processed for the ELISpot assay as described above. The number of IFN-γ positive spots in each well of the assay plate was measured using an Immunospot ELISpot reader system. Tumor tissue was also harvested and processed as described above. TIL phenotypes were analyzed by FACS analysis for % CD8⁺ TILS, % NK1.1⁺ TILs, CD8⁺ TILs EOMES expression, and CD8⁺ TILs degranulation.

Clinical signs (such as illness and health behavioral changes) were recorded for all animals once daily during the study using the body condition (BC) score system as previously described (Ullman-Cullere and Foltz, Lab Anim Sci. 1999; 49:319-23). Moribund mice were humanely euthanized by CO₂ asphyxiation. Body weights were recorded for all animals on study twice per week, including the termination day of each study. Tumors were measured with digital calipers in three dimensions for the duration of the experiment. Tumor volumes were calculated using the equation: Volume = 0.5236 (L × W × H); where L = length, W = width and H = height of the tumor. Kaplan-Meier survival curves were generated to quantify the interval of time from tumor inoculation to sacrifice and calculate the median survival time for each treatment group.

ELISpot assay was used to quantify the frequency of IFN-γ producing, P15E-specific CD8⁺ T cells was quantified by ELISpot assay. Immune phenotype of splenocytes and the tumor infiltrating lymphocytes (TILs) was performed by FACS (Fluorescence-activated cell sorting).

Statistical Analysis: Tumor volumes were measured twice per week throughout the study period. Tumor volume data was presented as the mean ± standard error of the mean (SEM). Tumor volume data was log transformed and two-way, repeated measures ANOVA with Tukey's correction for multiple comparisons was performed to measure statistical differences between treatment groups. Tumor weights were collected at study completion. The data was represented as the mean ± SEM. The T/C ratio was calculated as the tumor volume (or tumor weight) of the treatment group divided by the tumor volume (or tumor weight) of control group. Tumor weight data was evaluated with one-way ANOVA with Tukey's correction for multiple comparisons to measure statistical differences between treatment groups. The frequency of IFN-γ producing CD8⁺ T cells was quantified by ELISpot assay and represented as the mean number of spots per well (mean ± SEM). A one-way ANOVA with Tukey's correction for multiple comparisons was used for statistical analyses using GraphPad Prism Software. p<0.05 was determined to be statistically significant.

Study Design: Combination Therapy of Radiation with Anti-PD-L1/TGFβ Trap in MC38 Model in B-cell Deficient Mice to test abscopal effect. Group and treatment (N=6). Treatment started on day 0 with isotype control (400 µg, days 0, 2, 4), radiation (500 rads/day, days 0, 1, 2, 3), Anti-PD-L1/TGFβ Trap (164 µg, day 0), and Anti-PD-L1/TGFβ Trap (164 µg, day 0) + radiation (500 rads/day, days 0, 1, 2, 3). Radiation was applied only to the primary tumor, as shown in (Fig. 9A). Primary tumor volumes and secondary tumor volumes were measured twice weekly. Tumor volumes are presented as mean ± SEM.

### Results

Combination of Radiation with Anti-PD-L1/TGFβ Trap Demonstrated Synergistic Anti-tumor Efficacy. In an MC38 intramuscular tumor model (TI13-109), radiation (360 rads/day, day 0-3) or anti-PD-L1/TGFβ Trap monotherapy (55 or 164 µg, day 2) induced significant tumor growth inhibition (p<0.0001 respectively, vs. isotype control), whereas the combination of radiation and anti-PD-L1/TGFβ Trap induced remarkable therapeutic synergy compared to monotherapy with either radiation (p<0.0001) or anti-PD-L1/TGFβ Trap (p<0.0001) on day 10 (see FIG. 6A). The T/C ratio on day 14 based on the tumor weight was 0.45 for radiation therapy, 0.50 and 0.36 for anti-PD-L1/TGFβ Trap at 55 µg and 164 µg, respectively, and 0.04 vs. 0.01 for the radiation and anti-PD-L1/TGFβ Trap combination groups (55 µg vs. 164 µg, respectively) (see FIG. 6B). Tumor regression was observed, as early as 4 days after the anti-PD-L1/TGFβ Trap treatment, in 50% (10 out of 20) of the mice treated with anti-PD-L1/TGFβ Trap monotherapy, 100% (20 out of 20) of the mice treated with the combination therapy, and only 10% (1 out of 10) of the mice treated with radiation monotherapy. Furthermore, since anti-PD-L1/TGFβ Trap was given only as a single dose, 4 of 10 regressed tumors grew back in the anti-PD-L1/TGFβ Trap monotherapy group, whereas all the regressed tumors in the combination group continued to shrink up to day 14 when the mice were sacrificed for evaluation of immune function.

Immune Activation Was Correlated With the Antitumor Efficacy. On day 14, the mice were sacrificed and the frequency of IFN-γ producing, tumor-reactive (P15E) CD8⁺ T cells was quantified using an ex vivo ELISpot assay (see FIG. 6C). Only a moderate induction of IFN-γ producing tumor-reactive CD8⁺ T cells was observed in the radiation and anti-PD-L1/TGFβ Trap monotherapy group (p>0.05 and p<0.05 respectively, vs. isotype control). Consistent with the observed antitumor efficacy; however, mice treated with the combination therapy experienced a synergistic induction in the frequency P15E-specific, IFN-γ producing CD8⁺ T cells (see FIG. 6C). The CD8⁺ T cell IFN-γ production induced by combination therapy was 7-fold above that of the isotype control and at least 5-fold above those of the monotherapies (p<0.001 vs. each monotherapy, respectively). In this study (TI13-109), increasing the dose of anti-PD-L1/TGFβ Trap from 55 µg to 164 µg in the combination therapy did not further accelerate tumor regression. Due to a low CD8⁺ T cell yield in the high dose group, an adequate evaluation of the frequency of IFN-γ producing, tumor reactive (P15E) CD8⁺ T cells could not be performed. Therefore, a repeat study was performed to ensure the consistency of the findings.

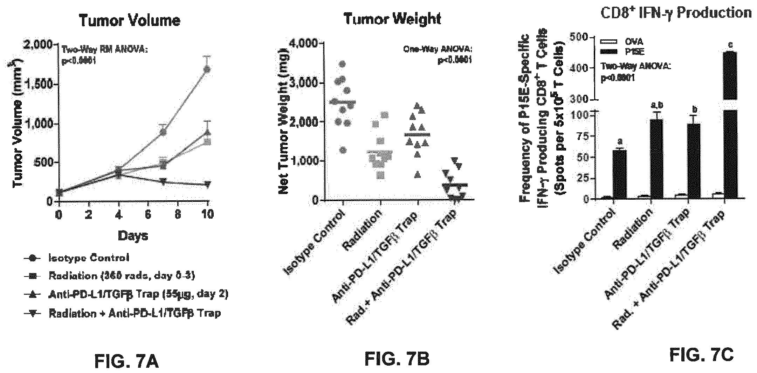

A repeat experiment (TI14-013) with the low dose of 55 µg of anti-PD-L1/TGFβ Trap yielded nearly identical synergistic effects on both tumor growth inhibition and induction of P15Especific CD8⁺ T cell IFN-γ production with the combination therapy (see Figure 7A-C).

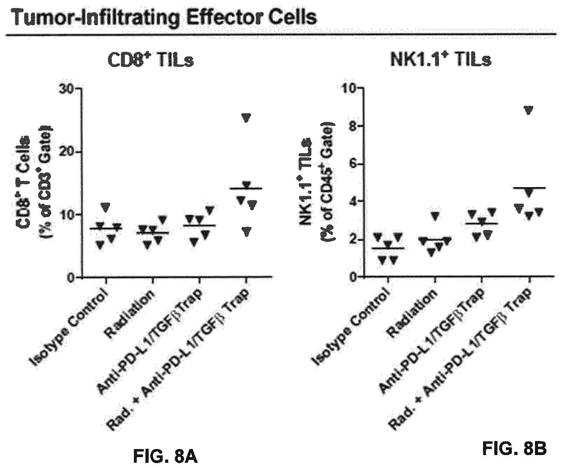

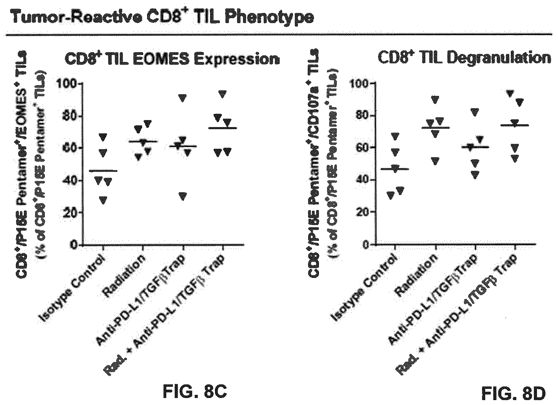

An analysis of the tumor-infiltrating lymphocytes of mice treated in study TI14-013 revealed elevations in the frequencies of CD8⁺ TILs and NK cells following treatment with the combination of radiation and anti-PD-L1/TGFβ Trap relative to either monotherapy or the Isotype control groups (see FIG. 8A-B). Additional analysis indicated that the combination of radiation and anti-PD-L1/TGFβ Trap therapy promoted the expression of the T-box transcription factor, Eomes, and degranulation (CD107a) on tumor-infiltrating CD8⁺ T cells (see FIG. 8C-D).

Combination therapy with radiation and a single dose of anti-PD-L1/TGFβ Trap reduced primary tumor volume relative to anti-PD-L1/TGFβ Trap or radiation alone (p<0.0001 for both, day 14) (Fig. 9B). However, combination therapy also reduced secondary tumor volume relative to anti-PD-L1/TGFβ Trap or radiation alone (p=0.0066 and p=0.0006, respectively, day 14) (Fig. 9C). Notably, neither radiation alone nor a single low dose of anti-PD-L1/TGFβ Trap significantly inhibited secondary tumor growth relative to isotype control treatment, indicating anti-PD-L1/TGFβ Trap synergized with radiation to induce an abscopal effect.

The observed synergies in antitumor efficacy and the frequencies of tumor-reactive, IFN-γ producing CD8⁺ T cells coupled with enhanced infiltration by effector CD8⁺ T cells and NK cells is consistent with the profound induction of innate and adaptive antitumor immune responses by combination therapy with radiation and the anti-PD-L1/TGFβ Trap molecule. As such, this therapeutic combination has clinically relevant applications for improving radiotherapy in cancer patients.

The data described herein demonstrate that standard of care external beam radiation therapy (EBRT)can be combined with anti-PD-L1/TGFβ Trap therapy to achieve synergistic tumor growth inhibition and the induction of tumor-reactive CD8⁺ T cell responses in a MC38 colorectal cancer model. As P15E is an endogenous retroviral antigen expressed by the MC38 tumor cell line (Zeh et al. J Immunol. 1999; 162:989-94), the observed increase in P15E-specific, IFN-γ producing CD8⁺ T cells constitutes a tumor-reactive, and not a generalized, T cell response following combination therapy. This synergistic immunological response is consistent with the enhanced antitumor efficacy observed in this therapeutic regimen, indicating that the combined treatment with radiation and anti-PD-L1/TGFβ Trap facilitates a CD8⁺ T cell mediated antitumor response. The combination of radiation and anti-PD-L1/TGFβ Trap therapy were also shown to promote MC38 tumor infiltration by CD8⁺ T cells and NK cells. Furthermore, the combination therapy induced an effector CD8⁺ TIL phenotype as evidenced by higher expression levels of the transcription factor, Eomes, and the degranulation marker, CD107a.

The observed results support the synergy of this combination strategy for potential clinical application. The sequential therapy of radiation and anti-PD-L1/TGFβ Trap can be of benefit to patients who have increased circulating TGFβ levels following radiotherapy. Furthermore, because of the strong synergistic effect observed even with a single low dose of anti-PD-L1/TGFβ Trap, a favorable safety profile in the clinic with such a combination therapy is expected.

### SEQUENCES

**SEQ ID NO: 1**
   Peptide sequence of the secreted anti-PD-L1 lambda light chain 
QSALTQPASVSGSPGQSITISCTGTSSDVGGYNYVSWYQQHPGKAPKLMIYDVSNRPSGVSNR
FSGSKSGNTASLTISGLQAEDEADYYCSSYTSSSTRVFGTGTKVTVLGQPKANPTVTLFPPSS
 
EELQANKATLVCLISDFYPGAVTVAWKADGSPVKAGVETTKPSKQSNNKYAASSYLSLTPEQW
KSHRSYSCQVTHEGSTVEKTVAPTECS
**SEQ ID NO: 2**
   Peptide sequence of the secreted H chain of anti-PDL 1 
EVQLLESGGGLVQPGGSLRLSCAASGFTFSSYIMMWVRQAPGKGLEWVSSIYPSGGITFYADT
VKGRFTISRDNSKNTLYLQMNSLRAEDTAVYYCARIKLGTVTTVDYWGQGTLVTVSSASTKGP
SVFPLAPSSKSTSGGTAALGCLVKDYFPEPVTVSWNSGALTSGVHTFPAVLQSSGLYSLSSVV
TVPSSSLGTQTYICNVNHKPSNTKVDKRVEPKSCDKTHTCPPCPAPELLGGPSVFLFPPKPKD
TLMISRTPEVTCVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSREEMTKNQVSLTCLVKGFYPS
DIAVEWESNGQPENNYKTTPPVLDSDGSFFLYSKLTVDKSRWQQGNVFSCSVMHEALHNHYTQ
KSLSLSPGK
**SEQ ID NO: 3**
   Peptide sequence of the secreted H chain of anti-PDL1/TGFβ Trap 
EVQLLESGGGLVQPGGSLRLSCAASGFTFSSYIMMWVRQAPGKGLEWVSSIYPSGGITFYADT
VKGRFTISRDNSKNTLYLQMNSLRAEDTAVYYCARIKLGTVTTVDYWGQGTLVTVSSASTKGP
SVFPLAPSSKSTSGGTAALGCLVKDYFPEPVTVSWNSGALTSGVHTFPAVLQSSGLYSLSSVV
TVPSSSLGTQTYICNVNHKPSNTKVDKRVEPKSCDKTHTCPPCPAPELLGGPSVFLFPPKPKD
TLMISRTPEVTCVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSREEMTKNQVSLTCLVKGFYPS
DIAVEWESNGQPENNYKTTPPVLDSDGSFFLYSKLTVDKSRWQQGNVFSCSVMHEALHNHYTQ
KSLSLSPGAGGGGSGGGGSGGGGSGGGGSGIPPHVQKSVNNDMIVTDNNGAVKFPQLCKFCDV
RFSTCDNQKSCMSNCSITSICEKPQEVCVAVWRKNDENITLETVCHDPKLPYHDFILEDAASP
KCIMKEKKKPGETFFMCSCSSDECNDNIIFSEEYNTSNPD
**SEQ ID NO: 4**
   DNA sequence from the translation initiation codon to the translation stop codon of the anti-PD-L1 lambda light chain (the leader sequence preceding the VL is the signal peptide from urokinase plasminogen activator) 
atgagggccctgctggctagactgctgctgtgcgtgctggtcgtgtccgacagcaagggcCAG
TCCGCCCTGACCCAGCCTGCCTCCGTGTCTGGCTCCCCTGGCCAGTCCATCACCATCAGCTGC
ACCGGCACCTCCAGCGACGTGGGCGGCTACAACTACGTGTCCTGGTATCAGCAGCACCCCGGC
 
AAGGCCCCCAAGCTGATGATCTACGACGTGTCCAACCGGCCCTCCGGCGTGTCCAACAGATTC
TCCGGCTCCAAGTCCGGCAACACCGCCTCCCTGACCATCAGCGGACTGCAGGCAGAGGACGAG
GCCGACTACTACTGCTCCTCCTACACCTCCTCCAGCACCAGAGTGTTCGGCACCGGCACAAAA
GTGACCGTGCTGggccagcccaaggccaacccaaccgtgacactgttcccccccatcctccgag
gaactgcaggccaacaaggccaccctggtctgcctgatctcagatttctatccaggcgccgtg
accgtggcctggaaggctgatggctccccagtgaaggccggcgtggaaaccaccaagccctcc
aagcagtccaacaacaaatacgccgcctcctcctacctgtccctgacccccgagcagtggaag
tcccaccggtcctacagctgccaggtcacacacgagggctccaccgtggaaaagaccgtcgcc
cccaccgagtgctcaTGA
**SEQ ID NO: 5**
   DNA sequence from the translation initiation codon to the translation stop codon (mVK SP leader: small underlined; VH: capitals; IgG1m3 with K to A mutation: small letters; (G4S)x4-G linker: bold capital letters; TGFβRII: bold underlined small letters; two stop codons: bold underlined capital letters) 
atggaaacagacaccctgctgctgtgggtgctgctgctgtgggtgcccggctccacaggcGAG
GTGCAGCTGCTGGAATCCGGCGGAGGACTGGTGCAGCCTGGCGGCTCCCTGAGACTGTCTTGC
GCCGCCTCCGGCTTCACCTTCTCCAGCTACATCATGATGTGGGTGCGACAGGCCCCTGGCAAG
GGCCTGGAATGGGTGTCCTCCATCTACCCCTCCGGCGGCATCACCTTCTACGCCGACACCGTG
AAGGGCCGGTTCACCATCTCCCGGGACAACTCCAAGAACACCCTGTACCTGCAGATGAACTCC
CTGCGGGCCGAGGACACCGCCGTGTACTACTGCGCCCGGATCAAGCTGGGCACCGTGACCACC
GTGGACTACTGGGGCCAGGGCACCCTGGTGACAGTGTCCTCCgctagcaccaagggcccatcg
gtcttccccctggcaccctcctccaagagcacctctgggggcacagcggccctgggctgcctg
gtcaaggactacttccccgaaccggtgacggtgtcgtggaactcaggcgccctgaccagcggc
gtgcacaccttcccggctgtcctacagtcctcaggactctactccctcagcagcgtggtgacc
gtgccctccagcagcttgggcacccagacctacatctgcaacgtgaatcacaagcccagcaac
accaaggtggacaagagagttgagcccaaatcttgtgacaaaactcacacatgcccaccgtgc
ccagcacctgaactcctggggggaccgtcagtcttcctcttccccccaaaacccaaggacacc
ctcatgatctcccggacccctgaggtcacatgcgtggtggtggacgtgagccacgaagaccct
gaggtcaagttcaactggtacgtggacggcgtggaggtgcataatgccaagacaaagccgcgg
gaggagcagtacaacagcacgtaccgtgtggtcagcgtcctcaccgtcctgcaccaggactgg
ctgaatggcaaggagtacaagtgcaaggtctccaacaaagccctcccagcccccatcgagaaa
accatctccaaagccaaagggcagccccgagaaccacaggtgtacaccctgcccccatcccgg
 
gaggagatgaccaagaaccaggtcagcctgacctgcctggtcaaaggcttctatcccagcgac
atcgccgtggagtgggagagcaatgggcagccggagaacaactacaagaccacgcctcccgtg
ctggactccgacggctccttcttcctctatagcaagctcaccgtggacaagagcaggtggcag
caggggaacgtcttctcatgctccgtgatgcatgaggctctgcacaaccactacacgcagaag
agcctctccctgtccccgggtgctGGCGGCGGAGGAAGCGGAGGAGGTGGCAGCGGTGGCGGT
GGCTCCGGCGGAGGTGGCTCCGGAatccctccccacgtgcagaagtccgtgaacaacgacatg
atcgtgaccgacaacaacggcgccgtgaagttccctcagctgtgcaagttctgcgacgtgagg
ttcagcacctgcgacaaccagaagtcctgcatgagcaactgcagcatcacaagcatctgcgag
aagccccaggaggtgtgtgtggccgtgtggaggaagaacgacgaaaacatcaccctcgagacc
gtgtgccatgaccccaagctgccctaccacgacttcatcctggaagacgccgcctcccccaag
tgcatcatgaaggagaagaagaagcccggcgagaccttcttcatgtgcagctgcagcagcgac
gagtgcaatgacaacatcatctttagcgaggagtacaacaccagcaaccccgacTGATAA
**SEQ ID NO: 6**
   Polypeptide sequence of the secreted lambda light chain of anti-PD-L1(mut)/ TGFβ Trap, with mutations A31G,D52E,R99Y 
QSALTQPASVSGSPGQSITISCTGTSSDVGGYNYVSWYQQHPGKAPKLMIYEVSNRPSGVSNR
FSGSKSGNTASLTISGLQAEDEADYYCSSYTSSSTYVFGTGTKVTVLGQPKANPTVTLFPPSS
EELQANKATLVCLISDFYPGAVTVAWKADGSPVKAGVETTKPSKQSNNKYAASSYLSLTPEQW
KSHRSYSCQVTHEGSTVEKTVAPTECS
**SEQ ID NO: 7**
   Polypeptide sequence of the secreted heavy chain of anti-PD-Ll(mut)/ TGFβ Trap 
EVQLLESGGGLVQPGGSLRLSCAASGFTFSMYMMMWVRQAPGKGLEWVSSIYPSGGITFYADS
VKGRFTISRDNSKNTLYLQMNSLRAEDTAIYYCARIKLGTVTTVDYWGQGTLVTVSSASTKGP
SVFPLAPSSKSTSGGTAALGCLVKDYFPEPVTVSWNSGALTSGVHTFPAVLQSSGLYSLSSVV
TVPSSSLGTQTYICNVNHKPSNTKVDKRVEPKSCDKTHTCPPCPAPELLGGPSVFLFPPKPKD
TLMISRTPEVTCVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSREEMTKNQVSLTCLVKGFYPS
DIAVEWESNGQPENNYKTTPPVLDSDGSFFLYSKLTVDKSRWQQGNVFSCSVMHEALHNHYTQ
KSLSLSPGAGGGGSGGGGSGGGGSGGGGSGIPPHVQKSVNNDMIVTDNNGAVKFPQLCKFCDV
RFSTCDNQKSCMSNCSITSICEKPQEVCVAVWRKNDENITLETVCHDPKLPYHDFILEDAASP
KCIMKEKKKPGETFFMCSCSSDECNDNIIFSEEYNTSNPD
**SEQ ID NO: 8**
   Human TGFβRII Isoform A Precursor Polypeptide (NCBI RefSeq Accession No: NP_001020018) 
MGRGLLRGLWPLHIVLWTRIASTIPPHVQKSDVEMEAQKDEIICPSCNRTAHPLRHINNDMIV
TDNNGAVKFPQLCKFCDVRFSTCDNQKSCMSNCSITSICEKPQEVCVAVWRKNDENITLETVC
HDPKLPYHDFILEDAASPKCIMKEKKKPGETFFMCSCSSDECNDNIIFSEEYNTSNPDLLLVI
FQVTGISLLPPLGVAISVIIIFYCYRVNRQQKLSSTWETGKTRKLMEFSEHCAIILEDDRSDI
SSTCANNINHNTELLPIELDTLVGKGRFAEVYKAKLKQNTSEQFETVAVKIFPYEEYASWKTE
KDIFSDINLKHENILQFLTAEERKTELGKQYWLITAFHAKGNLQEYLTRHVISWEDLRKLGSS
LARGIAHLHSDHTPCGRPKMPIVHRDLKSSNILVKNDLTCCLCDFGLSLRLDPTLSVDDLANS
GQVGTARYMAPEVLESRMNLENVESFKQTDVYSMALVLWEMTSRCNAVGEVKDYEPPFGSKVR
EHPCVESMKDNVLRDRGRPEIPSFWLNHQGIQMVCETLTECWDHDPEARLTAQCVAERFSELE
HLDRLSGRSCSEEKIPEDGSLNTTK
**SEQ ID NO: 9**
   Human TGFβRII Isoform B Precursor Polypeptide (NCBI RefSeq Accession No: NP_003233
**SEQ ID NO: 10**
   A Human TGFβRII Isoform B Extracellular Domain Polypeptide 
IPPHVQKSVNNDMIVTDNNGAVKFPQLCKFCDVRFSTCDNQKSCMSNCSITSICEKPQEVCVA
VWRKNDENITLETVCHDPKLPYHDFILEDAASPKCIMKEKKKPGETFFMCSCSSDECNDNIIF
SEEYNTSNPD
**SEQ ID NO: 11**
   (Gly₄Ser)₄Gly linker
   GGGGSGGGGSGGGGSGGGGSG
**SEQ ID NO: 12**
   Polypeptide sequence of the secreted heavy chain variable region of anti-PD-L1 antibody MPDL3280A
**SEQ ID NO: 13**
   Polypeptide sequence of the secreted light chain variable region of anti-PD-L1 antibody MPDL3280A and the anti-PD-L1 antibody YW243.55S70 
DIQMTQSPSSLSASVGDRVTITCRASQDVSTAVAWYQQKPGKAPKLLIYSASFLYSGVPSRFS
GSGSGTDFTLTISSLQPEDFATYYCQQYLYHPATFGQGTKVEIKR
**SEQ ID NO: 14**
   Polypeptide sequence of the secreted heavy chain variable region of anti-PD-L1 antibody YW243.55S70

EVQLVESGGGLVQPGGSLRLSCAASGFTFSDSWIHWVRQAPGKGLEWVAWISPYGGSTYYADS
VKGRFTISADTSKNTAYLQMNSLRAEDTAVYYCARRHWPGGFDYWGQGTLVTVSA

## Claims

1. A pharmaceutical composition for use in treating cancer in combination with radiation wherein the composition comprises:
a protein comprising;
(a) a human TGFβRII, or fragment thereof capable of binding TGFβ; and
(b) an antibody, or antigen-binding fragment thereof, binding human protein Programmed Death Ligand 1 (PD-L1) comprising
(i) a heavy chain variable region including an HVR-H1, an HVR-H2, and an HVR-H3 having the amino acid sequences of SYIMM, SIYPSGGITFYADTVKG and IKLGTVTTVDY, respectively, and
(ii) a light chain variable region including an HVR-L1, an HVR-L2, and an HVR-L3, having the amino acid sequences of TGTSSDVGGYNYVS, DVSNRPS, and SSYTSSSTRV, respectively,
wherein the protein used in combination with radiation of a primary tumor inhibits the growth of a secondary tumor or metastasis distal to the primary tumor treated with radiation.

2. A protein for use in treating metastatic cancer in combination with radiation
wherein the protein comprises;
(a) a human TGFβRII, or fragment thereof capable of binding TGFβ; and
(b) an antibody, or antigen-binding fragment thereof, binding human protein Programmed Death Ligand 1 (PD-L1) comprising
(i) a heavy chain variable region including an HVR-H1, an HVR-H2, and an HVR-H3 having the amino acid sequences of SYIMM, SIYPSGGITFYADTVKG and IKLGTVTTVDY, respectively, and
(ii) a light chain variable region including an HVR-L1, an HVR-L2, and an HVR-L3, having the amino acid sequences of TGTSSDVGGYNYVS, DVSNRPS, and SSYTSSSTRV, respectively,
wherein the protein used in combination with radiation of a primary tumor inhibits the growth of a secondary tumor or metastasis distal to the primary tumor treated with radiation.

3. The pharmaceutical composition for use of claim 1 or the protein for use of claim 2, wherein the antibody, or antigen-binding fragment thereof, that binds PD-L1 comprises amino acids 1-120 of SEQ ID NO: 2.

4. The pharmaceutical composition for use of claim 1 or the protein for use of claim 2, wherein the antibody, or antigen-binding fragment thereof, that binds PD-L1 comprises the amino acid sequence of SEQ ID NO: 2 except that the C-terminal lysine has been mutated to alanine.

5. The pharmaceutical composition for use of claim 1 or the protein for use of claim 2,, wherein the human TGFβRII, or fragment thereof capable of binding TGFβ comprises the amino acid sequence of SEQ ID NO: 10.

6. The pharmaceutical composition for use of claim 1 or the protein for use of claim 2, wherein the protein comprises the amino acid sequence of SEQ ID NO: 1 and SEQ ID NO: 3.

7. The pharmaceutical composition for use of claim 1 or the protein for use of claim 2, wherein the cancer or tumor is selected from the group consisting of colorectal, breast, ovarian, pancreatic, gastric, prostate, renal, cervical, myeloma, lymphoma, leukemia, thyroid, endometrial, uterine, bladder, neuroendocrine, head and neck, liver, nasopharyngeal, testicular, small cell lung cancer, non-small cell lung cancer, melanoma, basal cell skin cancer, squamous cell skin cancer, dermatofibrosarcoma protuberans, Merkel cell carcinoma, glioblastoma, glioma, sarcoma, mesothelioma, and myelodysplastic syndromes.

8. The pharmaceutical composition for use of claim 1 or the protein for use of claim 2, wherein the administration of an initial dose of radiation is followed by administration of the composition.

9. The pharmaceutical composition for use of claim 1 or the protein for use of claim 2, wherein multiple doses of radiation are administered.

10. The pharmaceutical composition for use of claim 1 or the protein for use of claim 2, wherein multiple doses of the composition are administered.

11. The pharmaceutical composition for use of claim 1 or the protein for use of claim 2, wherein the composition and one additional anti-cancer agent are administered sequentially.

## Patentansprüche

1. Pharmazeutische Zusammensetzung zur Verwendung bei der Behandlung von Krebs in Kombination mit Bestrahlung, wobei die Zusammensetzung Folgendes umfasst:
ein Protein, umfassend:
(a) einen menschlichen TGFβRII oder ein Fragment davon, der/das in der Lage ist, an TGFβ zu binden; und
(b) einen Antikörper oder ein antigenbindendes Fragment davon, der/das das menschliche Protein Programmed Death Ligand 1 (PD-L1) bindet, umfassend
(i) eine variable Schwerkettenregion, die eine HVR-H1, eine HVR-H2 und eine HVR-H3 mit den Aminosäuresequenzen SYIMM, SIYPSGGITFYADTVKG bzw. IKLGTVTTVDY umfasst, und
(ii) eine variable Leichtkettenregion, die eine HVR-L1, eine HVR-L2 und eine HVR-L3 mit den Aminosäuresequenzen TGTSSDVGGYNYVS, DVSNRPS bzw. SSYTSSSTRV umfasst,
wobei das Protein, das in Kombination mit Bestrahlung eines Primärtumors verwendet wird, das Wachstum eines Sekundärtumors oder von Metastasen distal zu dem mittels Bestrahlung behandelten Primärtumor verhindert.

2. Protein zur Verwendung bei der Behandlung von metastasierendem Krebs in Kombination mit Bestrahlung, wobei das Protein Folgendes umfasst:
(a) einen menschlichen TGFβRII oder ein Fragment davon, der/das in der Lage ist, an TGFβ zu binden; und
(b) einen Antikörper oder ein antigenbindendes Fragment davon, der/das das menschliche Protein Programmed Death Ligand 1 (PD-L1) bindet, umfassend
(i) eine variable Schwerkettenregion, die eine HVR-H1, eine HVR-H2 und eine HVR-H3 mit den Aminosäuresequenzen SYIMM, SIYPSGGITFYADTVKG bzw. IKLGTVTTVDY umfasst, und
(ii) eine variable Leichtkettenregion, die eine HVR-L1, eine HVR-L2 und eine HVR-L3 mit den Aminosäuresequenzen TGTSSDVGGYNYVS, DVSNRPS bzw. SSYTSSSTRV umfasst,
wobei das Protein, das in Kombination mit Bestrahlung eines Primärtumors verwendet wird, das Wachstum eines Sekundärtumors oder von Metastasen distal zu dem mittels Bestrahlung behandelten Primärtumor verhindert.

3. Pharmazeutische Zusammensetzung zur Verwendung nach Anspruch 1 oder Protein zur Verwendung nach Anspruch 2, wobei der Antikörper oder das antigenbindende Fragment davon, der/das PD-L1 bindet, die Aminosäuren 1-120 der SEQ ID NO: 2 umfasst.

4. Pharmazeutische Zusammensetzung zur Verwendung nach Anspruch 1 oder Protein zur Verwendung nach Anspruch 2, wobei der Antikörper oder das antigenbindende Fragment davon, der/das PD-L1 bindet, die Aminosäuresequenz der SEQ ID NO: 2 umfasst, außer dass das C-terminale Lysin zu Alanin mutiert wurde.

5. Pharmazeutische Zusammensetzung zur Verwendung nach Anspruch 1 oder Protein zur Verwendung nach Anspruch 2, wobei der menschliche TGFβRII oder ein Fragment davon, der/das in der Lage ist, TGFβ zu binden, die Aminosäuresequenz der SEQ ID NO: 10 umfasst.

6. Pharmazeutische Zusammensetzung zur Verwendung nach Anspruch 1 oder Protein zur Verwendung nach Anspruch 2, wobei das Protein die Aminosäuresequenz der SEQ ID NO: 1 und der SEQ ID NO: 3 umfasst.

7. Pharmazeutische Zusammensetzung zur Verwendung nach Anspruch 1 oder Protein zur Verwendung nach Anspruch 2, wobei der Krebs oder Tumor aus der aus Kolorektal-, Brust-, Eierstock-, Pankreas-, Magen-, Prostata-, Nieren-, Zervixkrebs, Myelomen, Lymphomen, Leukämie, Schilddrüsen-, Endometrium-, Uterus-, Blasen-, neuroendokrinem, Kopf-Hals-, Leber-, Nasopharynx-, Hodenkrebs, kleinzelligem Lungenkrebs, nicht kleinzelligem Lungenkrebs, Melanomen, Basalzellhautkrebs, Plattenepithelkrebs der Haut, Dermatofibrosarcoma protuberans, Merkelzellkarzinomen, Glioblastomen, Gliomen, Sarkomen, Mesotheliomen und myelodysplastischen Syndromen bestehenden Gruppe ausgewählt ist.

8. Pharmazeutische Zusammensetzung zur Verwendung nach Anspruch 1 oder Protein zur Verwendung nach Anspruch 2, wobei auf die Verabreichung einer anfänglichen Strahlendosis die Verabreichung der Zusammensetzung folgt.

9. Pharmazeutische Zusammensetzung zur Verwendung nach Anspruch 1 oder Protein zur Verwendung nach Anspruch 2, wobei mehrere Strahlendosen verabreicht werden.

10. Pharmazeutische Zusammensetzung zur Verwendung nach Anspruch 1 oder Protein zur Verwendung nach Anspruch 2, wobei mehrere Dosen der Zusammensetzung verabreicht werden.

11. Pharmazeutische Zusammensetzung zur Verwendung nach Anspruch 1 oder Protein zur Verwendung nach Anspruch 2, wobei die Zusammensetzung und ein weiteres Antikrebsmittel nacheinander verabreicht werden.

## Revendications

1. Composition pharmaceutique destinée à être utilisée dans le traitement du cancer en combinaison avec une irradiation, dans laquelle la composition comprend :
une protéine comprenant ;
(a) un TGFβRII humain, ou un fragment de celui-ci capable de se lier au TGFβ ; et
(b) un anticorps, ou un fragment de celui-ci se liant à l'antigène, qui se lie au ligand de la protéine de mort programmée 1 (PD-L1) humain comprenant
(i) une région variable de chaîne lourde comprenant une HVR-H1, une HVR-H2, et une HVR-H3 possédant les séquences d'acides aminés de SYIMM, SIYPSGGITFYADTVKG et IKLGTVTTVDY, respectivement, et
(ii) une région variable de chaîne légère comprenant une HVR-L1, une HVR-L2, et une HVR-L3, possédant les séquences d'acides aminés de TGTSSDVGGYNYVS, DVSNRPS, et SSYTSSSTRV, respectivement,
dans laquelle la protéine, utilisée en combinaison avec l'irradiation d'une tumeur primaire, inhibe la croissance d'une tumeur secondaire ou de métastases distales à la tumeur primaire traitée par irradiation.

2. Protéine destinée à être utilisée dans le traitement d'un cancer métastatique en combinaison avec une irradiation,
dans laquelle la protéine comprend ;
(a) un TGFβRII humain, ou un fragment de celui-ci capable de se lier au TGFβ ; et
(b) un anticorps, ou un fragment de celui-ci se liant à l'antigène, qui se lie au ligand de la protéine de mort programmée 1 (PD-L1) humain comprenant
(i) une région variable de chaîne lourde comprenant une HVR-H1, une HVR-H2, et une HVR-H3 possédant les séquences d'acides aminés de SYIMM, SIYPSGGITFYADTVKG et IKLGTVTTVDY, respectivement, et
(ii) une région variable de chaîne légère comprenant une HVR-L1, une HVR-L2, et une HVR-L3, possédant les séquences d'acides aminés de TGTSSDVGGYNYVS, DVSNRPS, et SSYTSSSTRV, respectivement,
dans laquelle la protéine utilisée en combinaison avec l'irradiation d'une tumeur primaire inhibe la croissance d'une tumeur secondaire ou de métastases distales à la tumeur primaire traitée par irradiation.

3. Composition pharmaceutique destinée à être utilisée selon la revendication 1 ou protéine destinée à être utilisée selon la revendication 2, dans laquelle l'anticorps, ou un fragment de celui-ci se liant à l'antigène, qui se lie à PD-L1 comprend les acides aminés 1-120 de SEQ ID NO: 2.

4. Composition pharmaceutique destinée à être utilisée selon la revendication 1 ou protéine destinée à être utilisée selon la revendication 2, dans laquelle l'anticorps, ou un fragment de celui-ci se liant à l'antigène, qui se lie à PD-L1 comprend la séquence d'acides aminés de SEQ ID NO: 2 à l'exception de la lysine C-terminale qui a été mutée en alanine.

5. Composition pharmaceutique destinée à être utilisée selon la revendication 1 ou protéine destinée à être utilisée selon la revendication 2, dans laquelle le TGFβRII humain, ou un fragment de celui-ci capable de se lier au TGFβ comprend la séquence d'acides aminés de SEQ ID NO: 10.

6. Composition pharmaceutique destinée à être utilisée selon la revendication 1 ou protéine destinée à être utilisée selon la revendication 2, dans laquelle la protéine comprend la séquence d'acides aminés de SEQ ID NO: 1 et SEQ ID NO: 3.

7. Composition pharmaceutique destinée à être utilisée selon la revendication 1 ou protéine destinée à être utilisée selon la revendication 2, dans laquelle le cancer ou la tumeur est sélectionné(e) dans le groupe consistant en le cancer colorectal, le cancer du sein, le cancer de l'ovaire, le cancer du pancréas, le cancer de l'estomac, le cancer de la prostate, le cancer du rein, le cancer du col de l'utérus, un myélome, un lymphome, une leucémie, le cancer de la thyroïde, le cancer de l'endomètre, le cancer de l'utérus, le cancer de la vessie, un cancer neuroendocrinien, le cancer de la tête et du cou, le cancer du foie, le cancer du nasopharynx, le cancer du testicule, le cancer du poumon à petites cellules, le cancer du poumon non à petites cellules, le mélanome, un cancer de la peau basocellulaire, un cancer de la peau épidermoïde, un dermatofibrosarcome protubérans, un carcinome à cellules de Merkel, un glioblastome, un gliome, un sarcome, un mésothéliome, et des syndromes myélodysplasiques.

8. Composition pharmaceutique destinée à être utilisée selon la revendication 1 ou protéine destinée à être utilisée selon la revendication 2, dans laquelle l'administration d'une dose initiale d'irradiation est suivie de l'administration de la composition.

9. Composition pharmaceutique destinée à être utilisée selon la revendication 1 ou protéine destinée à être utilisée selon la revendication 2, dans laquelle de multiples doses d'irradiation sont administrées.

10. Composition pharmaceutique destinée à être utilisée selon la revendication 1 ou protéine destinée à être utilisée selon la revendication 2, dans laquelle de multiples doses de la composition sont administrées.

11. Composition pharmaceutique destinée à être utilisée selon la revendication 1 ou protéine destinée à être utilisée selon la revendication 2, dans laquelle la composition et un agent anticancéreux supplémentaire sont administrés séquentiellement.
